# EUROPEAN PATENT SPECIFICATION

(11) **EP 3 975 843 B1**
(45) Date of publication and mention of the grant of the patent: **29.04.2026**
(21) Application number: 20728063.7
(22) Date of filing: 28.05.2020
(51) Int. Cl.: A61B 5/1455, A61B 5/1495, G01N 21/49, A61B 5/00

(54) **OPTICAL APPARATUS COMPRISING TWO SELF-CALIBRATED OPTICAL MEASUREMENT SETS**
OPTISCHE VORRICHTUNG MIT ZWEI SELBSTKALIBRIERTEN OPTISCHEN MESSSÄTZEN
APPAREIL OPTIQUE COMPRENANT DEUX ENSEMBLES DE MESURE OPTIQUE AUTO-CALIBRÉS

(30) Priority: 28.05.2019 EP 19177067
(43) Date of publication of application: 06.04.2022
(73) Proprietor: Universität Zürich, 8006 Zürich (CH)
(72) Inventor: KLEISER, Stefan, 79639 Grenzach-Wyhlen (DE); OSTOJIC, Daniel, 8046 Zürich (CH); WOLF, Martin, 8038 Zürich (CH)
(74) Representative: Mertzlufft-Paufler, Cornelius
(86) International application number: PCT/EP2020/064864
(87) International publication number: WO 2020/239922

(56) References cited:
- WO-A1-2019/077133
- WO-A2-02/12854
- US-A1- 2008 015 424
- US-A1- 2013 324 816
- JENNY C, BIALLAS M, TRAJKOVIC I, FAUCHERE JC, BUCHER HU, WOLF M: "Reproducibility of cerebral tissue oxygen saturation measurements by near-infrared spectroscopy in newborn infants", JOURNAL OF BIOMEDICAL OPTICS, vol. 16, no. 9, 097004, 2 September 2011 (2011-09-02), pages 1 - 6, XP060020428, ISSN: 1560-2281, DOI: 10.1117/1.3622756
- GUOQIANG YU ET AL: "Time-dependent blood flow and oxygenation in human skeletal muscles measured with noninvasive near-infrared diffuse optical spectroscopies", JOURNAL OF BIOMEDICAL OPTICS, 11 April 2005 (2005-04-11), United States, pages 024027, XP055242986, Retrieved from the Internet <URL:http://bioptics.engineering.uky.edu/files/2012/06/Guoqiang-Muscle-Paper_2005_JBO.pdf> [retrieved on 20160120], DOI: 10.1117/1.1884603
- PATTERSON M S ET AL: "ABSORPTION SPECTROSCOPY IN TISSUE-SIMULATING MATERIALS: A THEORETICAL AND EXPERIMENTAL STUDY OF PHOTON PATHS", APPLIED OPTICS, OPTICAL SOCIETY OF AMERICA, WASHINGTON, DC; US, vol. 34, no. 1, 1 January 1995 (1995-01-01), pages 22 - 30, XP000486126, ISSN: 0003-6935, DOI: 10.1364/AO.34.000022

## Description

The invention concerns an apparatus for measuring at least one optical or physiological parameter in a scattering medium, comprising a source set consisting of at least two light sources spaced from each other and each configured to emit a measurement wavelength, wherein said measurement wavelengths are substantially equal, a detector set consisting of at least two detectors spaced from each other and each configured to detect said measurement wavelengths after being backscattered by said medium, and an electronic unit configured to read-out said at least two detectors, wherein said at least two light sources and said at least two detectors are arranged on a measurement surface such that a first optical measurement set (OMS1) is formed, featuring two first source-detector-separations (SDS1a, SDS1b) substantially equal in size along with two second source-detector-separations (SDS2a, SDS2b) substantially equal in size and each larger than each of said first source-detector-separations (SDS2a or SDS2b > SDS1a or SDS1b).

The invention further concerns a method for obtaining a corrected deep penetration depth channel signal (cDPDC) from an apparatus according to the invention, which, as already mentioned, is designed for measuring an optical or physiological parameter in a scattering medium by illuminating the medium with at least one measurement wavelength and recording at least one intensity of backscattered light at the measurement wavelength with at least one detector of said apparatus.

Apparatuses with such features are already employed, for example for noninvasive in vivo optical absorption spectroscopy (see for example the scientific article *"*Time dependent blood flow and oxygenation in human skeletal muscles measured with noninvasive near-infrared diffuse optical spectroscopies", Journal of Biomedical Optics, 11 Paril 2005, by Guoqiang Yu et. al. or the publication WO 02/12854 A2), in particular for optical near-infrared-spectroscopy (NIRS) measurements or NIR neuromonitoring (NIN). With such apparatuses it is possible to determine arterial and venous oxygenation levels and, in particular, cerebral oxygenation, which is a highly important vital parameter. For this purpose, infrared light of a specific wavelength is emitted by one of the sources of the apparatus into the cerebral tissue, where the light is propagating by scattering. Part of the light is backscattered by the tissue and can be captured by a detector of the apparatus that measures the intensity of the back-scattered light. From such measured intensities, the wavelength specific light attenuation in the tissue can be determined, from which optical parameters such as wavelength specific absorption coefficients and physiological parameters such as oxygenation can be obtained. As an important example, once the absorption coefficient µa in a tissue has been determined at two or more wavelengths, oxy- and deoxy hemoglobin concentrations can be determined based on the Lambert-Beer law.

A major problem in such measurements is that the measurements signals obtained from deep tissue regions are highly attenuated. Hence, even small signal contaminations from various sources of error can render the measurement of deep tissue layers highly unreliable. On the other hand, in various applications, it is of utmost importance to obtain reliable and accurate measurement from deep tissue layers.

US 2013/324816 A1 proposes a method for calibrating detectors of a tissue oximetry device by emitting light into a tissue phantom with known optical properties and detecting said light with a plurality of detectors of the device. This calibration method can thus only be applied prior to a real measurement of human tissue.

US 2008/015424 A1, on the other hand, proposes a sort of self-calibration-scheme applied during an optical measurement to an apparatus which can measure tissue oxygenation optically. This calibration can minimize the detrimental influence of variables such as light scattering, absorption and other optical tissue properties.

The scientific article *"*ABSORPTION SPECTROSCOPY IN TISSUE-SIMULATING MATERIALS: A THEORETICAL AND EXPERIMENTAL STUDY OF PHOTON PATHS", Applied Optics, Optical Society Of America, vol.34, no.1, 1 January 1995, by Patterson et. Al. provides a diffusion model which allows calculation of the mean depth visited by detected photons for a given source-detector separation, which is important for sensor design.

WO 2019/077133 A1 describes a spectrophotometric apparatus, which can be designed as a NIRS sensor for measuring oxygenation in deep brain tissues and which employs light barriers for avoiding unwanted light piping in an optical layer forming between the skin and the sensor.

Finally, the scientific article *"*Reproducibility of cerebral tissue oxygen saturation measurements by near-infrared spectroscopy in newborn infants", Journal of Biomedical Optics, vo.16, no.9, 2 September 2011, by Jenny et. al. discloses an NIRS sensor with a sensor geometry which reduces the influence of inhomogeneities by employing two self-calibrating optical measurement sets, each featuring two sources and two detectors arranged at the corners of a rectangle.

Based on this background, it is a primary object of the present invention to provide an apparatus for measuring at least one optical or physiological parameter in a scattering medium, in particular cerebral tissue, which offers an improved measurement performance, in particular when measuring such parameters in deep layers of the medium / the tissue. Moreover, the invention aims at improving the usability of such an apparatus and at improving the security in detecting changes of oxygenation in deep tissue layers.

In accordance with the present invention, an apparatus is provided according to claim 1, which delivers such improved performance. In particular, the invention proposes an apparatus as introduced at the beginning, which, in addition, comprises a second optical measurement set (OMS2) which is formed on said measurement surface, featuring twc third source-detector-separations (SDS3a, SDS3b) substantially equal in size along with two fourth source-detector-separations (SDS4a, SDS4b) substantially equal in size and each larger than each of said third source-detector-separations (SDS4a or SDS4b > SDS3a or SDS3b). Furthermore, said fourth source-detector-separations are larger than said second source-detector-separations (SDS4a, SDS4b > SDS2a, SDS2b), and said electronic unit (11) is configured to evaluate measurement signals obtained from OMS1 and/or OMS2 in a self-calibrating-scheme, respectively. Such a self-calibrating-scheme has the technical effect that at least one variable measurement parameter, such as a light intensity of a source, a phase difference between light sources, an optical coupling efficiency, a temperature drift, a detector sensitivity, or the like, is eliminated.

Such an apparatus offers multiple specific advantages and possible configurations, as will become apparent below. In some configurations, the respective self-calibration may take into account signals from one OMS only (local measurement); in other it may take into account signals from both OMS (global measurement - c.f. below).

In a preferred embodiment, the electronic unit is configured to evaluate measurement signals obtained from said first optical measurement set (OMS1), in particular obtained from said first optical measurement set (OMS1) only, in a self-calibrating-scheme, and to evaluate measurement signals, obtained from said second optical measurement set (OMS2), in particular from said second optical measurement set (OMS2) only, in a self-calibrating-scheme. In this case, said electronic unit may be configured to output a self-calibrated low penetration depth channel (LPDC) signal obtained from OMS1, in particular obtained from OMS1 only, and a self-calibrated deep penetration depth channel (DPDC) signal obtained from OMS2, in particular obtained from OMS2 only, accordingly.

A major advantage of this concept for clinical NIRS use cases is that the LPDC-signal can be used to monitor short-term and transitional changes of the oxygenation and blood flow in superficial tissue layers. For example, when a patient enters a critical condition, the blood flow will typically first drop in outer tissue layers, while deep tissue layers will be supplied with enough blood flow for a longer time. Hence, a drop in oxygenation or blood flow monitored with the LPDC-signal can be considered as an early warning indicator, because in such a situation the DPDC signal may still show that blood flow/oxygenation in the deeper tissue layers is still sufficient (but is soon going to drop) and the user can react accordingly. This approach thus increases the safety of vital parameter monitoring for the patient. The separate self-calibration schemes are crucial for delivering two highly accurate and reliably measurement signals, which are largely immune to typical measurement variations of coupling efficiency or the like, and thus form the basis of such a early warning indication based on the self-calibrated LPDC signal. Without using a separate OMS for the LPDC signal that allows self-calibration, inhomogeneities such as liver spots on the skin or the like would easily render any signal obtained from superficial tissue layers highly unreliable, thus prohibiting an early warning mechanism.

Accordingly, the apparatus may be configured to output an early warning signal, in case a measurement quantity computed by the electronic unit from said scLPDC signal differs beyond a threshhold value from a measurement quantity computed by the electronic unit from said scDPDC signal. In such a case, the usability of the device can be further increased by configuring the apparatus such that it displays a chronological sequence of these two measurement quantities computed from said scLPDC signal and said scDPDC signal, respectively. This enables a user to monitor and compare both quantities in real-time and to act preventively, in case the patient is in danger of reaching a critical condition.

Following this concept more generally, said apparatus may be configured to output an early warning signal, in case the scLPDC and/or scDPDC and/or a measurement quantity computed by the electronic unit from said scLPDC and/or scDPDC signal differs beyond a threshold value, preferably beyond a threshold value computed by the electronic unit from said scDPDC and/or scDPDC signal. Furthermore, the apparatus may be configured to display a chronological sequence of said scLPDC signal and/or said scDPDC signal and/or the mentioned two measurement quantities computed from said scLPDC signal and said scDPDC signal, respectively.

In other words, in this case the apparatus offers two self-calibrated and separate measurement signals, namely the self-calibrated low penetration depth channel (scLPDC) signal and the self-calibrated deep penetration depth channel (scDPDC) signal, obtained with two separate optical measurement setups, namely with OMS1 and OMS2, respectively. For this purpose, the electronic unit may be configured to compute the scLPDC signal from output signals of the detectors belonging to OMS1 and to compute the scDPDC signal from output signals of the detectors belonging to OMS2. In addition, the electronic unit may be configured to evaluate the respective output signals for computing ratios of light intensities (which is equivalent to computing differences of Log-values of such intensities), for example, and to derive said parameter from such ratios.

Substantially equal in size may mean here with respect to different wavelengths or source-detector-separations that the variations are so small, that they can be neglected in the computation of the parameter to be measured with the apparatus. In other words, equal may be understood here as being limited by typical variations as resulting from inaccuracies of the placement of components or variations of the emissions wavelengths between different light sources employed in the apparatus.

For evaluating the measurement signals from OMS1 and OMS2, in particular the evaluation of the respective output signals of the detectors, respectively, in a self-calibration scheme, the electronic unit may be configured to apply an algorithm canceling out said at least one variable measurement parameter by taking into account the geometry of the arrangement of sources and detectors forming the respective OMS. Such canceling is possible in particular, if the sources and detectors, on which the algorithm relies, are symmetrically arranged on said measurement surface, in particular symmetrically within each of the two OMS, as will be explained in greater detail below (cf. also the explanations provided with respect to US 6,078,833 below).

As a result of the self-calibration scheme, the electronic unit can be configured to output the scLPDC signal and/or scDPDC signal independently from initial or temporal variations (e.g. drifts) of said at least one variable measurement parameter, in particular without prior calibration of the apparatus.

Each of the two OMS (OMS1 and OMS2) offers specific source-detector pairs (SDP), which each respective SDP defining a specific source-detector-separation (SDS), which may be the distance between the respective source and detector, as measured along said measurement surface.

Furthermore, OMS1 and OMS2 each offer a first pair of substantially equal SDS and a second pair of substantially equal SDS. In addition to this redundancy, in each of the two OMS, the respective second pair (SDS2a, SDS2b and SDS4a, SDS4b, respectively) offers a larger SDS as the respective first pair (SDS1a, SDS1b and SDS3a, SDS3b, respectively). And finally, OMS2 offers larger SDS as OMS1 (since SDS4a, SDS4b > SDS3a or SDS3b).

This very specific arrangement of light sources and detectors in combination with the very specific way of computing signals in a self-calibration scheme, based on output signals from the detectors of OMS1 and OMS2, respectively, allows operation of each of the two OMS as a self-calibrating optical circuit (SCOS) and hence recording of two self-calibrated signals from different depths of the medium, which may be in particular tissue.

The underlying reason for the latter property of the apparatus is that the larger SDS offered by OMS2 as compared to the SDS offered by OMS1 result in a larger mean photon-visit depth (MPVD), which can be understood, for example in the application case of in vivo absorption spectroscopy, as an average depth in the medium from which information is obtained. Hence, OMS2 is designed to optically probe deeper into said medium as compared to OMS1.

The optical properties of the apparatus can be modeled as follows: For a single point source (S) emitting photons into a uniform half-space of homogenous tissue limited by a plane (P) and a point detector (D), situated on the tissue surface in plane P at a certain SDS from the source S and collecting photons back-scattered by the tissue, the diffusion process in the tissue can be characterized by a single quantity, namely the effective attenuation coefficient µeff or its reciprocal, which is the penetration depth δ = 1/ µeff.

In consequence, the MPVD resulting from a specific SDS can be estimated by the empirical relationship MPVD = 0.5 (SDS δ)0.5 where SDS is the source-detector-distance between source S and detector D and δ is said penetration depth; δ can be readily calculated from the diffusion coefficient D = 1/[3(µa + µs)], based on material data for the absorption coefficient µa and the scattering coefficient µs of the tissue (which are attainable via optical measurements), as δ = 1/ µeff = 1 / (µa / D)0.5.

Thus, for a given scattering medium with known material data µa and µs, the necessary source-detector-distance SDS for a desired mean photon-visit depth MPVD can be accurately estimated, based on the above formula, as SDS = 4 MPVD / δ = 4 MPVD (µa / D)0.5 = 4 MPVD (3 µa (µa + µs) )0.5. In other words, by choosing a certain SDS in an absorption spectroscopy measurement scheme, it is possible to detect chromophores located in a specific depth in tissue.

Furthermore, as will be explained in greater detail below, for NIRS applications, in which said medium is tissue, in particular cerebral tissue, OMS1 can be designed to probe superficial layers of tissue, while OMS2 can be designed to probe deep layers of tissue. For example, the SDS of OMS1 may be less than 10 mm, thus excluding any significant contribution from deep layers to the measurement signal of OMS1.

Using the scLPDC signal from OMS1, it is therefore possible to remove those parts from the scDPDC signal, which result from superficial layers. This offers the advantage that a contamination of the scDPDC signal with dynamics in superficial tissue layers can be removed. This is particularly important since the sensitivity of a NIRS channel decreases exponentially with depth (i.e. with MPVD), such that NIRS-signals are generally biased towards superficial layers and hence easily affected by said superficial dynamics.

By using large SDS, it is possible to increase the sensitivity of a measurement channel to deeper tissue regions. However, at the same time, the signal-to-noise ratio (SNR) will be lower in the measured signal, because fewer photons will reach a respective detector per unit time without being absorbed. As a consequence, the scDPDC is highly susceptible to contamination from false signals originating from tissue layers at lower depth. The specific design of the apparatus addresses this problem, by providing an additional low penetration depth channel (LPDC) for correction of the scDPDC.

It is well known (see for example US 6,078,833, US 5661302 and US 8055321) that an OMS, comprising at least two spaced detectors and at least two spaced light sources, wherein the spacing between the at least two light sources and the at least two detectors is constrained to certain dimensions and wherein the light from the at least two sources in one measurement is of substantially identical wavelength (i.e. within certain tolerances) can be used as an SCOS. Signals received from said at least two detectors can be used to obtain data that are substantially independent of parameters such as the individual light intensity of the sources, the individual sensitivity of the detectors or various optical coupling efficiencies. Such an SCOS thus offers the benefit of delivering measurement data that is independent from initial or temporal variations of the above parameters. As is well known, with 2+ sources and 2+ detectors a self-calibrating algorithm can be used for cancelling out all instrumental and optical coupling factors.

For improving the robustness of the overall measurement, in particular the signal correction of the scDPDC as explained above, the invention proposes to employ each of the two OMS (i.e. OMS1 und OMS2) in a "self-calibrating-scheme" as just described. Accordingly, the electronic unit of the apparatus may be configured to perform such a self-calibrating algorithm on each of the two OMS (OMS1 and OMS2), respectively, based on the output signals received from the detectors of the respective OMS.

As a major advantage in comparison to the prior art, the apparatus according to the invention can offer such robustness for various depth, or - to be more precise - for various MPVD. For example, when using OMS1 of the apparatus in an oximeter measurement scheme and accidentally placing the light sources on a liver spot on the skin, the measurement is less prone to errors.

In summary, the invention proposes to use a self-calibrated low penetration depth channel (scLPDC) for mitigating the detrimental influence of superficial tissue layers on a self-calibrated deep penetration depth channel (scDPDC), measured in deep tissue layers using a larger SDS as compared to the SDS used in the LPDC. As a result, accurate and robust spectroscopic measurement of deep tissue layers become possible.

The major technical effect of the invention is that OMS1 can deliver an absolute value of said optical or physiological parameter, for example a concentration of a chromophore in a first depth from the measurement surface, whereas OMS2 can deliver a similar absolute value of said parameter but corresponding to a second depth from the measurement surface. Due to the specific choice of the SDS of each of the two OMS, the second depth optically probed by OMS2 is substantially larger than said first depth probed by OMS1. At the same time, each OMS can deliver an absolute measurement of light absorption in the respective depth in said medium, due to the specific geometry of the SDS in the respective OMS, based on a self-calibration measurement scheme, which requires no time-consuming calibration prior to a measurement and delivers a reliable measurement at the first shot. Hence, there is already a scLPDC signal from OMS1 which is highly useful for correcting the scDPDC signal from OMS2.

The present invention can be applied to any optical measurement apparatus which employs an optical measurement scheme based on light attenuation in a scattering medium. In particular, however, the invention may be advantageously applied to the field of noninvasive in vivo optical absorption, in particular NIR, spectroscopy for determining the concentration of endogenous (e.g. oxygenated or deoxygenated hemoglobin (HbO / HbR)) or exogenous chromophores (e.g. cells marked with optical markers) in tissue.

Another advantage of the apparatus presented herein is that an additional pulsoximeter functionality can be provided by employing the LPDC, which may offer an SDS in the range of 5 to 15 mm, as will be explained in greater detail below. Thus, the LPDC can deliver a pulsoximeter signal originating from superficial tissue layers. As OMS1 already offers several different light paths, the pulsoximeter measurement can be obtained from different tissue regions, thus rendering the measurement more reliable and repeatable. On the contrary, OMS2, which may feature SDS values of 25 mm or more, is less useful for pulsoximeter measurements, because of the lower SNR.

The computation described before may be done in particular by using a multi-wavelengths approach, in which the electronic unit is configured to evaluate measurement signals resulting from multiple wavelengths detected by the detectors of the apparatus.

For this purpose, the apparatus may feature several source sets, each consisting of at least two light sources spaced from each other and each configured to emit a measurement wavelengths, wherein said measurement wavelengths are substantially equal, and wherein the wavelengths offered by the source sets differ from each other, for example by at least 20 nm, preferably by at least 50 nm.

Alternatively, at least two broad-band light sources spaced from each other may be employed in the apparatus in combination with detectors which can selectively detect single measurement wavelengths, for example by using optical filters, or in combination with a spectrometer. Such an approach allows multi-wavelength measurements as well.

Furthermore, the wavelengths emitted by the sources of the apparatus may lie within the NIR, i.e. between 780 nm and 2500 nm. This way, the apparatus can be configured for NIRS (NIR spectroscopy) applications.

It is further noted here that the electronic unit may be a single component or consist of multiple components. For example, according to a specific configuration, the apparatus may comprise a mobile electronic device, for example a tablet. This device may be connected by a cable or a wireless connection to a measurement head of the apparatus. In such cases, a first component of the electronic unit may be located inside the measurement head while a second component of the electronic unit may be provided by the mobile electronic device.

According to the invention, there exist further advantageous embodiments solving the afore mentioned problem, which are listed in the sub-claims and described in the following:
Depending on the desired application, resulting target measurement depth and desired signal processing, said first source-detector-separations may be shorter than or substantially equal to said third source-detector-separations (SDS1a, SDS1b <= SDS3a, SDS3b). In this case, the short SDS of OMS2 will be at least as large as the short SDS of OMS1. Therefore, OMS2 will probe more deeply into tissue than OMS1.

Moreover, the third source-detector-separations may be substantially equal to said second source-detector-separations (SDS3a, SDS3b ≈ SDS2a, SDS2b) or the third source-detector-separations may be substantially longer than said second source-detector-separations (SDS3a, SDS3b > SDS2a, SDS2b). In these cases, there will be no overlap between the depth ranges probed by OMS1 and OMS2.

In other applications, the third source-detector-separations may be substantially shorter than said second source-detector-separations (SDS3a, SDS3b << SDS2a, SDS2b). In this case, the depth ranges probed by OMS1 and OMS2 will overlap. In consequence, a common signal part shared by the scLPDC and scDPDC will be larger as compared to the case of no overlap.

Another useful design rule for particular application cases proposes that the sum of said fourth and third source-detector-separations is larger or equal than the sum of said second and first source-detector-separations (SDS4a + SDS3a >= SDS2a + SDS1a).

Another useful design rule for a purely linear arrangement is that the fourth source-detector-separations are each larger or equal than the sum of the third, second, and first source-detector-separations (SDS4a >= SDS3a + SDS2a + SDS1a).

The invention further includes a concept of using "inner OMS", which will now be explained:
As laid out in claim 1, the two optical measurement sets OMS1 and OMS2 offered by the apparatus each feature at least two spaced detectors and these two spaced detectors of OMS1, for example denoted as D2 and D3, lie within an area enclosed by a circle. The diameter of said circle is a distance between at least two spaced detectors of OMS2, for example denoted as D1 and D4, and the center of the circle is equidistant from said at least two spaced detectors of OMS2 (i.e. D1 and D4) such that OMS1 forms an inner OMS with respect to OMS2.

Alternatively or additionally, as laid out in the 2^{nd} alternative of claim 1, OMS1 and OMS2 may each feature at least two spaced sources. In this case, the at least two spaced sources of OMS1, for example denoted as S2 and S3, lie within an area enclosed by a circle whose diameter is a distance between at least two spaced sources of OMS2 (for example denoted as S1 and S4) and whose center is equidistant from said at least two spaced sources of OMS2, such that OMS1 forms an inner OMS with respect to OMS2.

Finally, as described in claim 1, OMS1 and OMS2 each feature at least two spaced sources or at least two spaced detectors and at least two spaced sources or detectors of OMS1 lie within an area enclosed by a circle whose diameter is a distance between at least two spaced sources or detectors of OMS2 and whose center is equidistant from said at least two spaced sources or detectors of OMS2, such that OMS1 forms an inner OMS with respect to OMS2.

Common to all those approaches is that OMS1 is located such that a first region in said medium optically probed by OMS1 lies within a second region probed by OMS2. Such an arrangement implies, that the scLPDC and scDPDC signals recorded by OMS1 and OMS2, respectively, share a common signal part that originates from chromophores located in the first region. By contrast, if no such arrangement is used, the scLPDC and scDPDC siganl may share a part that emanates from the same depth layer within the tissue but not the same location. The concept of using an inner OMS thus ensures, that the scLPDC signal is representative for a part of the scDPDC signal that results from the first region, lying within the second region.

Another advantage of using the concept of "inner OMS" is that coupling efficiencies can be canceled by the self-calibration algorithm in both OMS1 and OMS2.

The concept of using an "inner OMS" may alternatively be described with respect to overlaps of the respective source-detector-separations (SDS) of OMS1 and OMS2: Following the concept, the SDS 1a, 1b, and 2a, 2b of the inner (and smaller) OMS1 should show a full overlap with at least one of the SDS of the outer (and larger) OMS2. This may be achieved by following the design rule that the fourth SDS should be larger than the sum of the second and first SDS (SDS4a > SDS1a + SDS2a), at least with respect to the respective projection of the SDS on the longitudinal axis.

In greater detail, according to the invention, OMS1 and OMS2 may each comprise a source sub-set and each source sub-set may consist of at least two light sources spaced from each other. Furthermore, OMS1 and OMS2 may each comprise a detector sub-set and each detector sub-set may consist of at least two detectors spaced from each other.

According to the invention, which simplifies the self-calibration algorithms applied to the measurement signals obtained from OMS1 and OMS2, the sources and detectors forming the respective OMS1 and OMS2, respectively, are each linearly arranged on a respective longitudinal axis. In other words, all light sources and detectors defining the SDS of the respective OMS1 or OMS2 may be arranged on a longitudinal axis. As will become apparent from the different design examples illustrated in the figures, the longitudinal axes of OMS1 and OMS2 either coincide (i.e. only one common longitudinal axis resulting in an overall linear arrangement) or share at least a common center (e.g. in case the two longitudinal axis are arranged in an X-formation, resulting in an overall non-linear arrangement).

Part of those detectors or sources may be shared by both OMS. For example, in a typical arrangement according to the invention, OMS1 and OMS2 may share two common detectors and each of the two OMS may feature its own pair of two separate light sources. In yet another typical arrangement according to the invention, OMS1 and OMS2 may share two common light sources, for example denoted as S1 and S2, and each of the two OMS may feature its own pair of two separate detectors, for example OMS1 may feature two separate detectors denoted as D2 and D3 and OMS2 may feature two separate detectors denoted as D1 and D4.

Sharing of light sources and detectors among the two OMS may be actual of benefit in terms of cost, energy consumption or size of the apparatus. In order to guarantee, that the desired self-calibration scheme can still be applied, the at least two light sources of said source sub-sets and/or the at least two detectors of said detector sub-sets may be each symmetrically arranged to a common global symmetry line.

In particular, if the two OMS share a common SDS, for example in case SDS2a, SDS2b = SDS3a, SDS3b, such an arrangement is also of advantage for performing a self-calibration that takes into account signals from both OMS (global measurement). For example, in case the superficial layers are homogenous, and the resulting light attenuation occurring for the common SDS is comparable for different source-detector-pairs, variations of measurement parameters occurring for the sources and/or detectors of OMS1 and OMS2 can be eliminated. As a result, a self-calibrated measurement signal representing an optical path probed by the first source-detector-separations (SDS1a and SDS1b) and a self-calibrated measurement signal representing an optical path probed by the fourth source-detector-separations (SDS4a and SDS4b) can be obtained.

Simply set, the electronic unit may thus be configured to output a self-calibrated global measurement signal (scGMS) obtained from both OMS1 and OMS2. Moreover, in case OMS1 and OMS2 share a common SDS, in particular if SDS2a, SDS2b = SDS3a, SDS3b, said scGMS may comprise a self-calibrated measurement signal representing an optical path probed by the first source-detector-separations (SDS1a and SDS1b) and a self-calibrated measurement signal representing an optical path probed by the fourth source-detector-separations (SDS4a and SDS4b). In addition, such an approach reduces the demands for the required dynamic range of single detectors of the apparatus, because each detector can be designed for a narrow detection range.

Furthermore, it is preferable for performing signal processing taking into accounts self-calibrated signals obtained from OMS1 and OMS2, when at least one source-detector-pair defining one of said two second source-detector-separations (SDS2a or SDS2b) and at least one source-detector-pair defining one of said two third source-detector-separations (SDS3a or SDS3b) are symmetrically arranged to a respective common local symmetry line.

It is further of particular advantage, if OMS1 and/or OMS2 feature two separate of such common local symmetry lines, respectively. In a very specific arrangement, each of these two symmetry lines may run through one of the two common light sources.

As will now be explained, the technical advantages of the apparatus according to the invention may be advantageously applied to clinical applications in the field of NIR spectroscopy.

As was already indicated before, the measurement wavelengths emitted by said at least two light sources of the apparatus may lie in the NIR-range, i.e. between 780 nm and 2500 nm.

For example, according to one embodiment, the first source-detector-separations (SDS1a, and SDS1b), preferably and said second source-detector-separations (SDS2a, and SDS2b), may be chosen such that in a clinical NIRS measurement situation, the corresponding mean photon-visit depth (MPVD) of SDS1a, and SDS1b, and preferably also of SDS2a, and SDS2b, lies outside of cerebral tissue of a typical subject, for which said apparatus is designed to be used. As was explained previously, the MPVD can be estimated precisely, based on known material data of the tissue under investigation and the respective SDS of the measurement channel used. In addition, depending on the subject, for example a neonate or an adult, the skull and outer tissue layers may be more or less thicker, such that the depth of the cerebral tissue may differ. With such an arrangement, it is possible to measure non-cerebral tissue only using OMS1 and to subtract these signal portions from the DPDC.

Accordingly, it is of advantage in such a situation, if the fourth source-detector-separations (SDS4a, and SDS4b) are chosen such that the corresponding MPVD lies inside of said cerebral tissue. This is because in this case, OMS2 can be used to accurately measure a scDPDC from deep cerebral tissue layers.

For obtaining a useful scLPDC signal of superficial tissue layers under investigation with OMS2 when examining an adult person with the apparatus, it is proposed that the second source-detector-separations (SDS2a, SDS2b) and/or the first source-detector-separations (SDS1a, SDS1b) are shorter than 12 mm, most preferably shorter than 10 mm. In such a situation, it makes particular sense, if the fourth source-detector-separations (SDS4a, SDS4b) are longer than 20 mm, most preferably longer than 25 mm, because such values allow recording of cerebral (i.e. non-superficial) tissue layers of an adult. Signal components resulting from superficial tissue in the head of the adult can then be regressed from the scDPDC signal obtained from OMS2 by taking into account the scLPDC signal obtained from OMS1.

On the other hand, for obtaining a useful scLPDC siganl of superficial tissue layers under investigation with OMS2 when examining an infant with the apparatus, it is proposed that the second source-detector-separations (SDS2a, SDS2b) and/or the first source-detector-separations (SDS1a, SDS1b) are shorter than 5 mm, most preferably shorter than 3 mm. In such a situation it makes particular sense, if the fourth source-detector-separations (SDS4a, SDS4b) are longer than 15 mm, most preferably longer than 20 mm, because such values allow recording of cerebral (i.e. non-superficial) tissue layers of an infant. As above, signal components resulting from superficial tissue in the head of the infant can then be regressed from the scDPDC signal obtained from OMS2 by taking into account the scLPDC signal obtained from OMS1.

For clinical applications, the following combination of ranges for the different SDS used in the apparatus may be of particular advantage:
the first source-detector-separations (SDS1a and SDS1b) are between 1 and 15 mm;
the second and third source-detector-separations (SDS2a, SDS2b, SDS3a, SDS3b) are between 10 mm and 25 mm; and
the fourth source-detector-separations (SDS4a, SDS4b) are between 20 mm and 65 mm.

For convenient use of the OMS offered by the apparatus, the electronic unit may be configured to drive and control the at least two light sources configured to emit the measurement wavelengths. In particular, the electronic unit may be configured to address said at least two detectors and/or said at least two light sources through a multiplexer.

The apparatus may further comprise an output unit configured to output, for example acoustically and/or optically, a first measurement value derived from said scDPDC obtained from OMS2 and measured in a deep region of said medium. Such a first measurement value may be in particular a first concentration of a chromophore, e.g. HbO, in said medium or a first tissue oxygen saturation (StO2)-value.

In this case, it is preferable if the output unit is also configured to output a second measurement value derived from the scLPDC signal obtained from OMS1 and measured in a superficial region of said medium, located between said deep region and said measurement surface. Such a second measurement value may be, in particular, a second concentration of said chromophore in the medium or for example a second StO2-value.

Such an output unit may be, for example, provided by a tablet (forming a part of the apparatus) connected to a sensor head of the apparatus.

Even more of advantage for clinical use is an embodiment in which the output unit is configured to output, for example acoustically and/or optically, preferably along with said first measurement value, a first MPVD-value displaying an mean photon visit depth (MPVD) probed by OMS2. Preferably, the output unit may be further configured to output, in particular along with said second measurement value, a second MPVD-value displaying an MPVD probed by OMS1. The respective MPVDs can be computed by the electronic unit, based on known material data of the medium under investigation and the SDS of the respective OMS employed.

Furthermore, the apparatus may comprise a computing unit, which may be part of said electronic unit, and which may be configured to regress superficial components from the scDPDC signal obtained from OMS2 by processing the scDPDC signal and the scLPDC signal obtained from OMS1 and to deliver a corrected deep penetration depth channel (cDPDC) signal that is substantially free of optical noise arising from superficial layers optically probed by OMS1.

The electronic unit may thus be further configured to compute a corrected measurement value of said parameter from the cDPDC signal. The corrected measurement value may then be transferred to a tablet or other mobile electronic device to be visualized and/or further processed.

In other words, an output unit of the apparatus, in particular the output unit described above, may be configured to output, for example acoustically and/or optically, a corrected measurement value of said parameter computed from the cDPDC signal, in particular by the electronic unit.

In yet another embodiment, the apparatus may comprise a computing unit, which may be said computing unit explained before, and which may be configured to compute a physiological parameter from said scLPDC signal obtained from said OMS1. Such a physiological parameter may be, for example, a concentration of a chromophore, for example HbO, or a pulse rate or respiratory rate.

The computation of such parameters may be achieved, in particular, by applying a pulse oximetry measurement scheme. Thus, the apparatus can be used as a pulse oximeter and as an oximeter for recording cerebral oxygenation levels at the same time.

For the purpose of additional pulse oximeter measurements, the apparatus may feature an analog or digital filter configured to separate an AC-component from the scLPDC signal and said computing unit may be configured to compute a temporal variation of said concentration from said AC-component. Alternatively or additionally, the apparatus may feature an amplifier with an adjustable frequency transfer function, for example by way of a control voltage, and said computing unit may be configured to adjust said frequency transfer function to separate an AC-component from an amplified signal outputted by said amplifier and to compute said concentration from said AC-component of said amplified signal.

For broad usability in clinical applications, the source set of the apparatus may emit not only two substantially equal wavelengths but at least two, preferably four, most preferably eight different measurement wavelengths.

In this case, it is of advantage if said at least two, preferably four, most preferably eight different measurement wavelengths are separated from each other by at least 10 nm, preferably by at least 20 nm, respectively, because this allows the calculation of said parameter on a broader data basis. Particularly, said at least two, preferably four, most preferably eight different measurement wavelengths may also comprise at least one wavelength outside of the NIR-range, for example in the UV-VIS-range, i.e. between 100 nm and 780 nm, and/or beyond 800 nm, in particular beyond 830 nm.

Generally, the SDS of the apparatus may be spaced from each other, respectively. However, in particular embodiments, some of the SDS may overlap when projected along said measurement surface.

This may be particularly the case, if said first source-detector-separations and/or said second source-detector-separations and/or said third source-detector-separations and/or said fourth source-detector-separations are linearly arranged along a common longitudinal axis of the apparatus. Such a design is of advantage since an overlap of the SDS along the measurement surface corresponds in an overlap of the regions in the medium optically probed by the respective SDP defining the respective SDS, which is of advantage for the measurement scheme proposed herein.

In one particular embodiment, the first and second source-detector-separations defining OMS1 overlap at least with the fourth source-detector-separations of OMS2. In this case, OMS2 may thus form an outer OMS, and OMS1 may form an inner OMS, in the sense explained above.

For clinical applications, it may be of further advantage if the measurement surface, on which the source set and detector set of the apparatus are arranged, is bendable. For easier bending, the measurement surface may even show a pre-defined curvature in a relaxed equilibrium state.

Finally, the apparatus may comprise a housing surrounding at least one of the at least two light sources and at least one of the at least two detectors. In addition, the at least one light source and the at least one detector surrounded by said housing may be mounted on a common printed circuit board at a distance of less than 2 mm from each other, thus allowing a highly compact design, that allows realization of ultra-short SDS without sacrificing optical or electrical performance. For further improving the optical and electrical performance, said housing may form a slit aperture for limiting angles at which light can be emitted from the at least one light source surrounded by said housing and/or the housing may provide an electrical shielding against electromagnetic interference (EMI) .

In accordance with the present invention, there is also provided a method, which solves the afore-mentioned problem. In particular there is provided a method as introduced at the beginning, which involves using the apparatus of the present invention, further comprising the following steps of:
- acquiring a low penetration depth channel (LPDC) signal with a first optical measurement setup OMS1 offering two pairs of substantially equal source-detector-separations (1a/1b and 2a/2b), wherein the two pairs of the OMS1 differ in their SDS values,
- acquiring a deep penetration depth channel signal (DPDC) with a second optical measurement setup OMS2 offering two pairs of substantially equal SDS (3a/3b and 4a/4b), wherein the two pairs of the OMS2 differ in their SDS values, and wherein OMS2 offers larger SDS values as OMS1,
- applying a self-calibration algorithm on the LPDC signal and the DPDC, respectively, to obtain a self-calibrated LPDC (scLPDC) signal and a self-calibrated DPDC (scDPDC) signal, and
- regressing signal components of the scLPDC signal from the scDPDC signal to obtain a corrected deep penetration depth channel (cDPDC) signal.

Such regression may be achieved by appropriate signal processing of the scLPDC signal and scDPDC signal. As a result, the cDPDC signal can be substantially free of optical noise that originates from superficial tissue layers, which are optically probed by OMS1. As has been explained before, OMS1 is less sensitive to deep layers and highly sensitive to superficial layers due to the shorter SDS, as compared to OMS2. At the same time, said optical noise can be present in both the scLPDC and the scDPDC signal, because OMS1 and OMS2 may be arranged such that they optically probe the same superficial layers. Hence, the scLPDC signal can comprise said optical noise to be removed from the scDPDC signal.

This method delivers the same increased measurement performance and advantages previously outlined with respect to the apparatus according to the invention. Ideally, an apparatus, on which this method is applied, may be in particular according to one of the claims directed towards an apparatus and/or as previously explained.

Preferred embodiments of the present invention shall now be described in more detail, although the present invention is not limited to these embodiments , but rather defined by the appended patent claims

With reference to the accompanying drawings, where features with corresponding technical function are referenced with same numerals even when these features differ in shape or design:
- Fig. 1: is a view of an apparatus according to the invention, namely an NIR-oximeter,
- Fig. 2: shows a detailed view of the distal end of the lower side of the apparatus of Fig. 1,
- Fig. 3: illustrates a schematic cross-sectional side view of the apparatus of Fig. 1 when being placed on the skull of neonate,
- Fig. 4: illustrates the specific arrangement of light sources and detectors on the measurement surface offered by the apparatus of Fig. 1,
- Fig. 5: is a top view on the arrangement of light sources and detectors of Fig. 4,
- Fig. 6: is an alternative arrangement of light sources and detectors with respect to the design of Fig. 4,
- Fig. 7: is a top view on the arrangement of light sources and detectors of Fig. 6,
- Fig. 8-11: are illustrations of possible designs of an apparatus according to the invention,
- Fig. 12: shows a top view on a non-linear arrangement of light sources and detectors of an apparatus according to the invention, and
- Fig. 13: shows yet another possible design of an apparatus according to the invention.

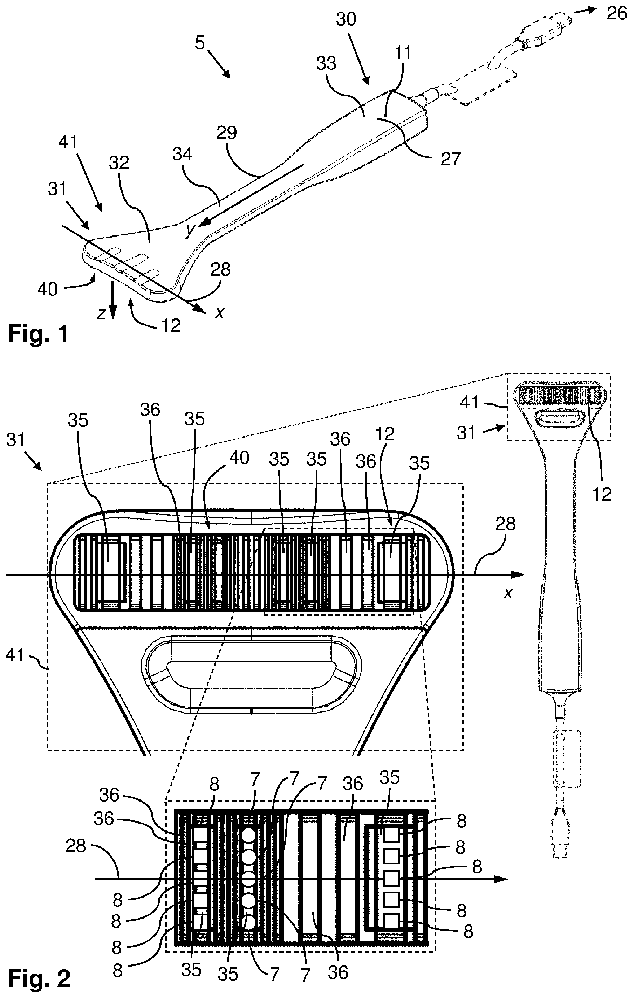

Figure 1 shows an apparatus 5 according to the invention, namely an oximeter for performing non-invasive cerebral NIRS diagnostics of neonates. For this purpose, a measurement surface 12 on the lower side of the sensor head 41 at the distal end 31 of the apparatus 5 is brought into skin contact on the head of a neonate. To ease this skin contact, the sensor head 41 and its measurement surface 12 are bendable and show a pre-defined curvature in a relaxed equilibrium state.

The apparatus 5 features a flexible hull 29 made from a soft silicone tube. At the distal end 31, a flexible measurement printed circuit board 32 carrying light sources 7 and detectors 8 is placed inside of the hull 29; at the proximal end 30, a stiff printed circuit board carrying an electronic unit 11 for control of the light sources 7 and detectors 8 is arranged inside of the hull 29. The electronic unit 11 is linked to the detectors 8 and light sources 7 via a flat cable enveloped by the hull 29 and not visible in figure 1.

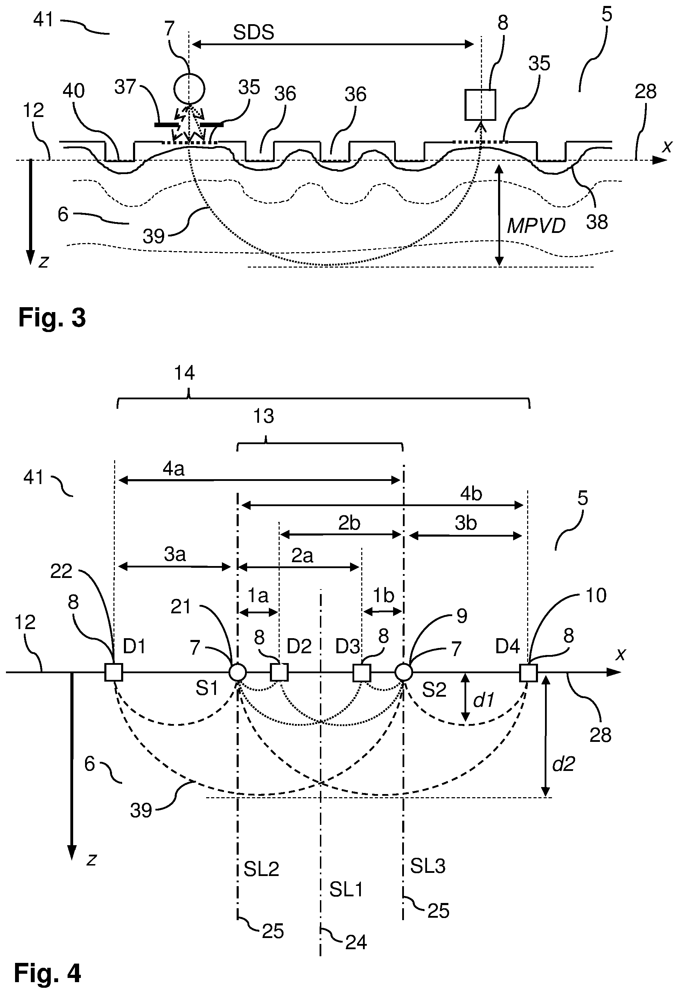

The lower side of the apparatus 5 of figure 1 with the measurement surface 12 is depicted on the right side of figure 2. Figure 2 also displays a detailed view of the lower side of the sensor head 41 revealing windows 35 which are formed in a contact surface 40 to be brought into skin contact. Through the windows 35 light is emitted and received by the apparatus 5. In between the windows 35, elongated optical barriers 36 are placed, whose rectangular cross-section is schematically illustrated in figure 3.

As is further visible in the small lower inset of figure 2, behind the windows 35 multiple sources 7 each emitting a measurement wavelength are placed as well as detectors 8 for receiving the light after being back-scattered by the skull of the neonate, i.e. by the scalp, skull, and cerebral tissue. To enable a compact design and a bendability of the measurement surface at the same time, all of the light sources 7 and detectors 8 are mounted on common measurement printed circuit board 32. Moreover, some sources 7 and detectors 8 show a distance of less than 2 mm from each other and are surrounded by a housing, which provides electrical shielding against electromagnetic interference as well as an optical slit aperture 37 (c.f. figure 3).

During a measurement, the sensor head 41 of the apparatus 5 is placed with its contact surface 40 onto the skin 38 on the skull of a neonate to be diagnosed, as schematically depicted in figure 3. As is visible in figure 3, the skin may penetrate in between the optical barriers 36 such that optical crosstalk by light piping between the light source 7 and the detector 8 shown in figure 3 is suppressed.

The light source 7 in figure 3 emits a measurement wavelength through an optical slit aperture 37 and a window 35 into the skull of the neonate, which is the scattering medium 6 to be probed optically and non-invasively by the apparatus 5.

Due to the scattering in the various tissue layers, indicated by curved dashed lines in figure 3, an average path that photons travel through the medium 6 to reach the detector 8 resembles the mean optical path 39 indicated by a dotted banana-shaped line in figure 3. This path 39 also defines a mean photon visit depth, with is denoted as MPVD in figure 3, and which characterizes a depth in the tissue from which information is optically retrieved by the source detector pair (SDP) shown in figure 3. As has been explained in detail above, the MPVD is a direct function of the distance between the corresponding SDP, which is denoted as source-detector-separation or SDS and measured along a measurement surface 12 offered by the sensor head 41, on which the light sources 7 and detectors 8 are arranged. In figure 3, this measurement surface 12 coincides with the contact surface 40 of the sensor head 41.

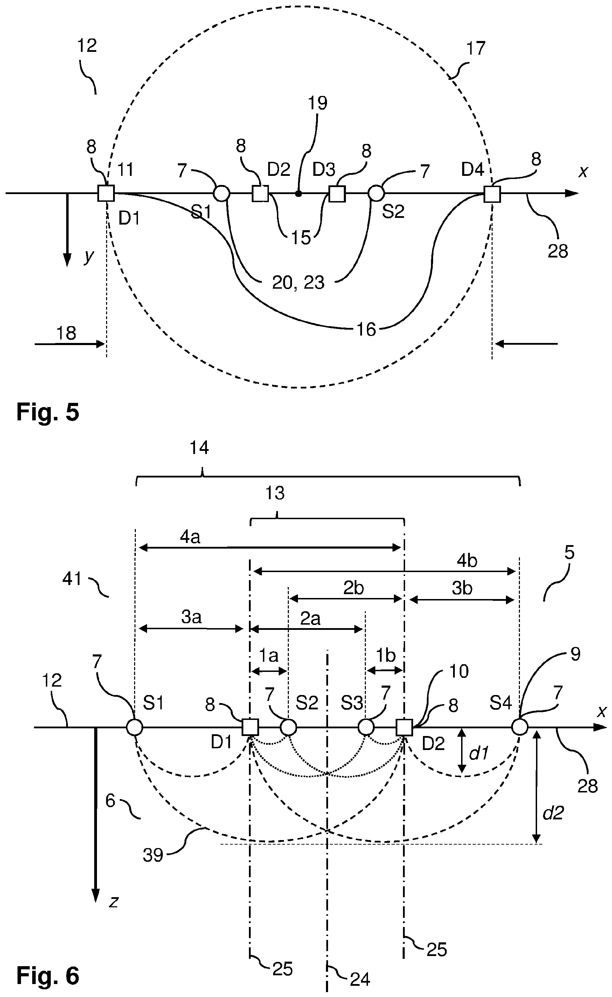

Figure 4 is a schematic illustration of the specific arrangement of the light sources 7 and detectors 8 on the measurement surface 12 offered by the sensor head 41 of the apparatus 5. Shown is a schematic cross-section through the sensor head 41, with the z-axis (c.f. figure 1) indicating the direction in which light is emitted into the tissue, as was already illustrated in figure 3. Each of the light sources S1 and S2 schematically represented by a small circle is actually made up of a total of 5 different LEDs emitting each an NIR-measurement wavelength. These LEDs are indicated in the lower inset of figure 2 by circles each denoted with a reference numeral 7. Likewise, each of the detectors D1, D2 in figure 6 can stand for a number of detectors located at the respective position on the longitudinal axis 28.

As depicted from figure 4, the apparatus 5 thus features at least two light sources 7, denoted schematically as S1 and S2, which are arranged on a longitudinal axis 28 running in the measurement surface 12 (c.f. figure 1). S1 and S2 are spaced from each other by 20 mm, each submit a common measurement wavelength in the NIR-range (i.e. between 780 nm and 2500 nm) and thus form a source set 9. The source set 9 offers a total of eight different NIR-measurement wavelengths, separated by more than 20 nm from each other, respectively.

Furthermore, the apparatus 5 offers four detectors 8, denoted as D1, D2, D3 and D4 in figure 4, which are likewise arranged along the longitudinal axis 28. Each of the four detectors D1, D2, D3 and D4 can detect light at the measurement wavelength emitted from S1 or S2, after the light has traveled through the tissue 6. The four detectors 8 thus form a detector set 10.

The arrangement in figure 4 is such that a first source-detector pair (SDP), formed by S1 and D3, defines a source-detector-separation (SDS) 1a of 5 mm and another first SDS 1b is defined by S2 and D3, which is also 5 mm long.

Accordingly, a relatively small MPVD results, as indicated by the dotted banana shaped lines between S1 and D2 and S2 and D3, respectively, which illustrate the corresponding mean optical paths 39 of photons emitted by S1 and S2 and received by D2 and D3, respectively.

In the same manner, further SDP and corresponding SDS are defined as follows:
a second SDS 2a between S1 and D3 of 15 mm;
another second SDS 2b between S2 and D2 of 15 mm;
a third SDS 3a between S1 and D1 of 15 mm;
another third SDS 3b between S2 and D4 of 15 mm;
a fourth SDS 4a between S2 and D1 of 35 mm;
another fourth SDS 4b between S1 and D4 of 35 mm;

The electronic unit 11 of the apparatus 5 is configured to address the light sources 7 and detectors 8 such that each of the above SDPs can be measured individually. This is possible for example by time multiplexing or by using frequency encoding techniques. For this purpose, the apparatus may feature a multiplexer through which the electronic unit 11 can address the individual light sources 7 and detectors 8.

As is illustrated by the dotted banana shaped curves in figure 4, S1, D2, D3, and S2 form a first optical measurement set 13 (OMS1), which features two first SDS of 5 mm each, namely 1a and 1b, and two second SDS of 15 mm each, namely 2a and 2b. As is illustrated by the dashed banana shaped curves in figure 4, D1, S1, S2, and D4 form a second optical measurement set 14 (OMS2), which features two third SDS of 15 mm each, namely 3a and 3b, and two fourth SDS of 35 mm each, namely 4a and 4b. From these values, it is clear that the two second SDS 2a and 2b are each larger than each of the first SDS 1a and 1b; furthermore the two fourth SDS 4a and 4b are each larger than each of the third SDS 3a and 3b.

At the same time, the two fourth SDS 4a and 4b of OMS2 14 are each larger than each of the second SDS 2a and 2b of OMS1 13; likewise the two first SDS 1a and 1b of OMS1 13 are each smaller than each of the third SDS 3a and 3b of OMS2 14. Furthermore the two third SDS 3a and 3b are equal in size to the two second SDS 2a and 2b.

The advantage of this specific arrangement is that OMS1 13 is designed to probe superficial layers of the medium 6, whereas OMS2 14 is designed to probe deep layers, as is readily visible by comparing the different MPVDs of the two OMS, denoted as d1 and d2 in figure 4.

In order to benefit from this arrangement, the electronic unit 11 of the apparatus 5 is configured to evaluate signals received from the detectors D2 and D3 of OMS1 13 in a self-calibrating-scheme, which benefits from the fact that each of the two SDS offered by OMS1 13, namely the first and second SDS 1a and 2a, is redundant, because of the existence of the SDS 1b and 2b. This allows application of known self-calibration algorithms by the electronic unit 11 on the output signals received from D2 and D3 to remove variable measurement parameters such as, for example, variations in the light intensities emitted by the sources S1 and S2 or variations in the sensitivity of the detectors D2 and D3.

In the same manner, the electronic unit 11 is further configured to evaluate signals received from the detectors D1 and D4 of OMS2 14 in a self-calibrating-scheme. The redundancy between the SDS 3a and 3b and 4a and 4b is readily apparent from figure 4. As a result of this design, the electronic unit can compute and output a self-calibrated low penetration depth channel (scLPDC) signal derived from the output signals of the detectors D2 and D3 of OMS1 13 and a self-calibrated deep penetration depth channel (scDPDC) signal derived from the output signals of the detectors D1 and D4 of OMS2 14.

As is visible from figure 5, which presents a top view on the arrangement of light sources 7 and detectors 8 on the measurement surface 12 (xy-plane - c.f. figure 1), OMS2 14 features two detectors spaced from each other 16, namely D1 and D4, and OMS1 13 features two such spaced detectors 15, namely D2 and D3. As indicated by the circle 17, whose center is equidistant from D1 and D4 and whose diameter 18 is equal to the distance from D1 to D4, D2 and D3 lie within an area enclosed by said circle 17. As is obvious from the combined views of figures 4 and 5, OMS1, i.e. S1, D3, D3 and S2, forms an inner OMS with respect to OMS1, because the region probed by OMS1 (indicated by the dotted curves in Fig. 4) lies within a larger and deeper region optically probed by OMS2 (indicated by the dashed curves in Fig. 4).

In other words, the tissue regions which are probed by OMS1 and OMS2 partly overlap in the xz-plane, since the banana shaped curves only illustrate mean photon paths and the real tissue volume illuminated by the respective source is larger than this mean photon path (since the photons are propagating by scattering in zig-zag lines).

Another detail apparent from Figure 6 is that the respective second and fourth source-detector-separations (SDS) 2a, 2b and 4a, 4b overlap in the xy-plane, respectively. Overlapping hence not merely means having the same source 7 or detector 8, because, for example, 4a and 4b do not share a common source 7 or detector 8.

Moreover, corresponding second and fourth SDS overlap, i.e. 4a overlaps with 2a and 4b with 2b. Finally, there is an overlap between the SDS forming a respective OMS, i.e. 4a overlaps with 3a, and 2b overlaps with 1b, as examples.

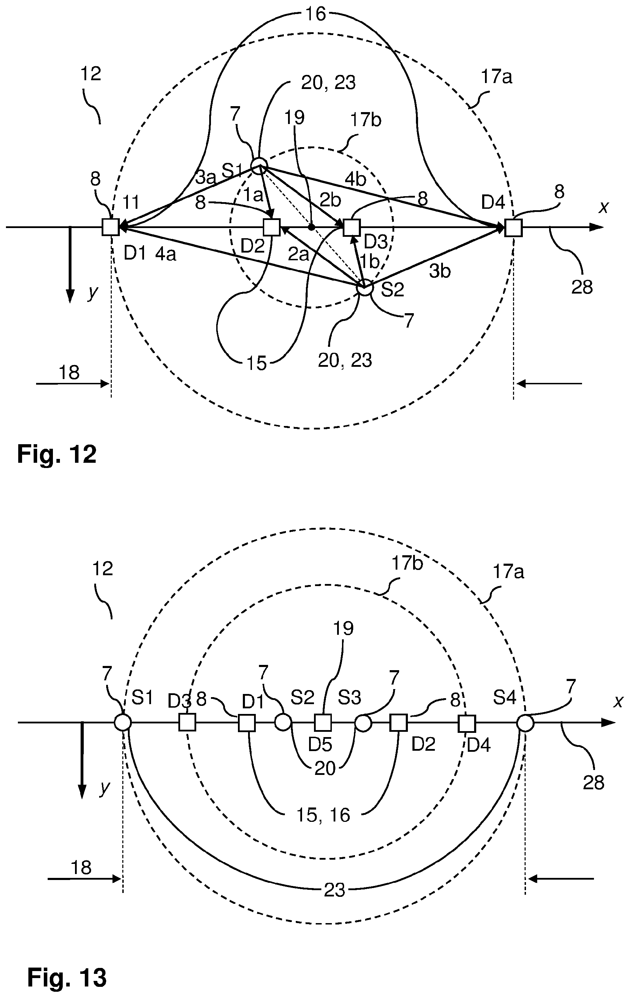

These observations are also true for the (overall) non-linear design according to figure 12, at least for the respective projections of the respective SDS onto the longitudinal x-axis 28. The result of this design rule ("respective second and fourth source-detector-separations (SDS) 2a, 2b and 4a, 4b show an overlap in their projections onto a longitudinal axis 28") is that the tissue regions optically probed by these pairs of second and fourth SDS 2a, 2b and 4a, 4b show some overlap, which is beneficial for the self-calibration scheme proposed herein.

Another key feature of the optical probe designs proposed herein is readily apparent from figure 4: No two of the source-detector-separations 1a, 1b, 2a, 2b, 3a, 3b, 4a and 4b extend between the same source-detector-pair (e.g. 1a is defined between S1 and D2, while 1b is defined between S2 and D3).

All of the above mentioned features are universally applicable features that may be used in other arrangements of light sources 7 and detectors 8 as well.

The optical designs displayed in the figures share some other common features: For example, as may be understood by comparing figures 1-4, the first and second OMS1 13 and OMS2 14 are arranged on the same contact or measurement surface of the apparatus 5, which is brought into skin contact during a measurement. Moreover, optical elements (either common light sources 7 or common detectors 8) may be shared between OMS1 13 and OMS2 14. Preferably, these shared optical elements are inner elements (not outermost) of the specific arrangements. For example, in the design according to figure 4, inner sources S1 and S2 are shared by OMS1 and OMS2 while in the design of figure 6, inner detectors D1 and D2 are shared by OMS1 and OMS2. This approach minimizes the number of elements required for implementing the technical advantages provided by the invention and also safeguards, that coupling efficiencies can be canceled out by the self-calibration performed by the apparatus.

As a more general design rule, optical arrangements may be preferred in which the fourth SDS 4a is at least a factor of 1.5 larger than the second SDS 2a (SDS4a > 1.5 SDS2a). Another useful design rule is that the sum of the fourth and third SDS should be a factor of 1.5 larger than the second and first SDS (SDS4a + SDS3a > 1.5 (SDS2a + SDS1a)). To ensure that the tissue regions probed by OMS1 and OMS2 truly differ, another useful design rule is that the fourth SDS should be generally larger than two times the second SDS (SDS4a > 2.0 SDS2a).

Figure 6 shows an alternative arrangement which may be used in the apparatus 5: in this specific design, the geometry is identical to the design illustrated in Fig. 4 except that the locations of light sources 7 and the locations of detectors 8 have been exchanged such that the apparatus 5 features four light sources 7 and two detectors 8. In other words, OMS1 13 is now made up from D1, S2, S3 and D2, whereas S1, D1, D2 and S4 form OMS2 14.

The design of figure 6 can be used in the same manner as described before in which the electronic unit 11 may derive a scLPDC signal from OMS1 13 and a scDPDC signal from OMS2 14. What has changed is the direction of light propagation which is for example from left to right from S1 to D1 in figure 6 and not from right to left from S1 to D1 as was the case in figure 4. The mean optical paths 39 have remain unchanged however, which shows that light sources 7 and detectors 8 are interchangeable in the concept according to the invention.

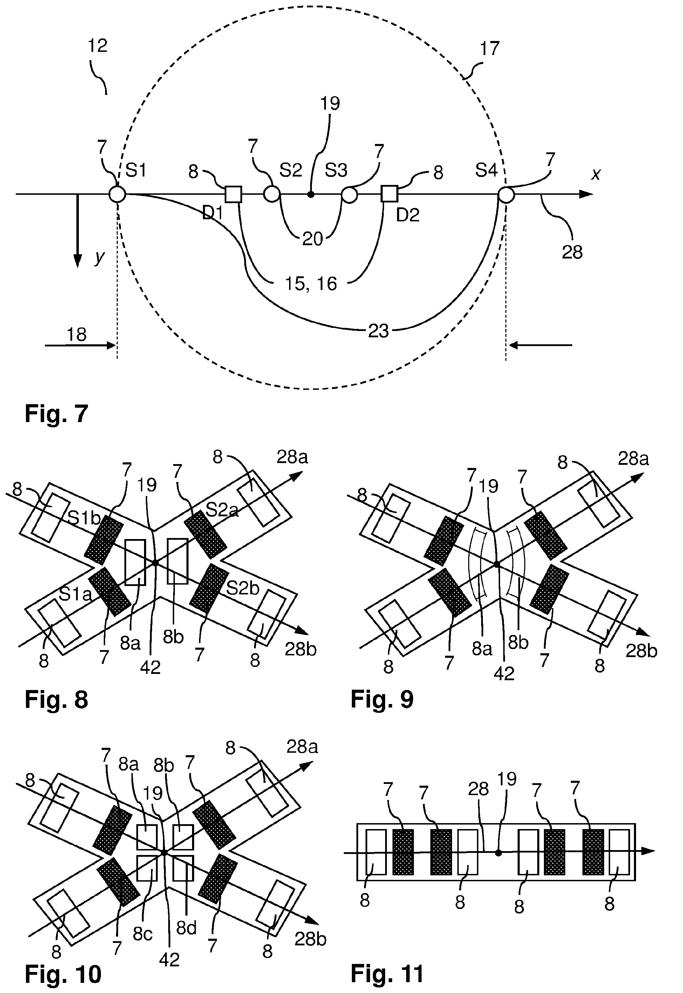

Figure 7 illustrates a top view on the arrangement according to figure 6. As visible, the circle 17 previously described with respect to figure 5 is now defined by the outer spaced light sources 23, namely S1 and S4, which belong to OMS2 14. The two spaced detectors 15, namely D1 and D2 as well as the two spaced sources 20, namely S2 and S3, of OMS1 13, respectively, all lie within the circle 17. In consequence, in the design according to figure 6 OMS1 13 forms an inner OMS with respect to OMS2 14.

From figure 4 it is further obvious that a source sub-set 21, consisting of the two light sources S1 and S2, which belongs to both OMS1 13 and OMS2 14, is symmetrically arranged to the global symmetry line 24, denoted as SL1 in figure 4 and running through the center of the arrangement. Furthermore, a first detector sub-set 22, consisting of detectors D1 and D4 and belonging to OMS2 14, and a second detector sub-set 22, consisting of detectors D2 and D3 and belonging to OMS1 13, are also each symmetrically arranged to the global symmetry line 24.

In addition, detector D1 of OMS2 14 and detector D3 of OMS1 13 are symmetrically arranged to a first common local symmetry line 25 denoted as SL2. SL2 runs through light source S1 shared by OMS1 and OMS2. Likewise, detector D4 of OMS2 14 and detector D3 of OMS1 13 are symmetrically arranged to a second common local symmetry line denoted as SL3. SL3 runs through light source S2 shared by OMS1 and OMS2. In other words, the source detector pair (SDP) D1-S1 and the SDP S1-D3 are each symmetrically arranged with respect to SL2, whereas the SDPs S2-D2 and S2-D4 are symmetrically arranged with respect to SL3. As has been explained in greater detail above, this symmetry is advantageous for obtaining a self-calibrated global measurement signal (scGMS) obtained from both OMS1 and OMS2.

With a maximum SDS of 15 mm, OMS1 13 is designed to probe into superficial, mostly non-cerebral tissue, when the apparatus is placed on the head of an adult. By contrast, the maximum SDS of 35 mm offered by OMS2 14 is designed to optically probe deeply into cerebral tissue, because the MPVD denoted as d2 in figure 4 is much larger than the MPVD offered by OMS1 and denoted as d1. As a result, the electronic unit 11 may regress signal components from superficial tissue layers in the scDPDC signal obtained with OMS2 14, based on the scLPDC signal from OMS1 13.

The apparatus 5 of figure 1 can be linked with an USB-cable to an output unit 26 in the form of a tablet (not shown in Figure 1), which displays a first measurement value of StO2 measured with OMS2 14 in the deep tissue region indicated by depth d2 in figure 4 as well as a second measurement value of StO2 measured with OMS1 13 in the superficial tissue region indicated by depth d1 in figure 4.

Said first value is actually a corrected measurement value of StO2, as the electronic unit 11 has computed this value based on a corrected deep penetration depth channel (cDPDC) signal, which was obtained by processing the scDPDC signal from OMS2 14 and the scLPDC signal from OMS1 13. The cDPDC signal is substantially free of optical noise arising from superficial layers optically probed by OMS2, because this noise has been recorded by OMS1 and is part of the scLPDC signal, which has been used by the electronic unit 11 for regressing these signal components form the original scDPDC signal obtained from OMS2. For this regression, it is of great advantage that OMS1 actually forms an inner OMS with respect to OMS2, as was previously discussed, because the superficial layers optically probed by OMS1 and OMS2 are to a large part the same.

Additionally, the tablet displays the corresponding MPVD values, i.e. the depth optically probed by OMS1 and OMS2, respectively, such that the user of the apparatus 5 can assign the displayed StO2-values to a certain measurement depth. Furthermore, a computing unit 27 of the electronic unit 11 computes a concentration of HbO and resulting oxygenation levels in a pulse oximetry measurement scheme from the scLPDC signal, and the output unit displays a corresponding pulse frequency. This additional oximetry functionality is possible because of the very short SDS offered by OMS1 13. For extracting the pulse frequency from the measured scLPDC signal, the apparatus 5 further features a digital filter which separates an AC-component from the scLPDC signal, such that the computing unit 27 can compute the temporal variation of the concentration of HbO.

In the designs according to figures 4 and 5, as well as 6 and 7, respectively, all four SDS 1a/1b, 2a/2b, and 3a/3b, 4a/4b are linearly arranged along the common longitudinal axis 28, which runs in the direction of the measurement printed circuit board 32 inside the sensor head 41, as illustrated in figure 1. Moreover, the first and second SDS 1a/1b, 2a/2b overlap with the fourth SDS 4a/4b, as can be seen from the horizontal arrows in figures 4 and 6, illustrating these SDS.

Figure 12 illustrates another possible design of an apparatus which is not covered by the claimed invention. The view of Figure 12 is similar to that of Figure 5 in that four detectors D1, D2, D3 and D4 are still arranged on a longitudinal axis 28 and symmetrically with respect to the center 19 of the arrangement. However, the light sources S1 and S2 are no longer placed on the longitudinal axis 28 but arranged off-axis, resulting in an overall non-linear arrangement of light sources 7 and detectors 8. More accurately, sources S1 and S2 are still arranged symmetrically to the center 19, as indicated by circle 17b. As a result, source S1 offers identical SDS towards the detectors 8 as source S2. Hence, here again, two separate measurement sets OMS1 and OMS2 (13 and 14) can be defined and employed according to the invention.

As in Figure 5, the electronic unit may thus evaluate signals received from detectors D1 and D4 of OMS2 14 in a self-calibrating-scheme. Again, the redundancy between the SDS 3a and 3b and 4a and 4b (illustrated by the dark arrows) is readily apparent from figure 12. As a result of this non-linear design of Figure 12, the electronic unit can compute and output a self-calibrated low penetration depth channel (scLPDC) signal derived from the output signals of the detectors D2 and D3 of OMS1 13 and a self-calibrated deep penetration depth channel (scDPDC) signal derived from the output signals of the detectors D1 and D4 of OMS2 14. Finally figures 8-11 illustrate top views on possible other arrangements of light sources 7 and detectors 8, which all enable the formation of an apparatus 1 according to the invention which can produce a self-calibrated low penetration depth channel (scLPDC) signal obtained from an OMS1 13, and self-calibrated deep penetration depth channel (scDPDC) signal obtained from OMS2 14. All four arrangements of figures 8-11 show a central symmetry center point 19.

Figure 11 illustrates a fully linear arrangement, similar to the design of figure 4 but with two additional light sources 7.

In the designs of figures 8-10, there are two separate longitudinal axes 28a and 28b, and each linear arrangement along the respective axis 28a or 28b offers a subset of at least two light sources 7 and four detectors 8 linearly and symmetrically arranged on the respective axis 28a or 28b, similar to the design illustrated in figure 5 (i.e. each linear arrangement offers two inner sources S1 and S2). Of course, as can be seen by comparing figures 5 and 7, detectors and sources may be exchanged in such designs, which is also applicable to the designs according to figures 8-11.

The two longitudinal axes 28a and 28 b in figures 8-10 are arranged in an X-formation featuring an intersection point 42. This intersection point 42 coincides with the center 19 of the arrangement.

Visible in the examples of figures 8, 9 and 10 are also two or four inner detectors 8a, 8b, 8c, and 8d, respectively, which are symmetrically arranged with respect to center 19 and surrounded by four light sources 7 (e.g. S1a, S2a, S1b, S2b in figure 8), respectively.

In the designs of figures 8-10, each arrangement of light sources 7 and detectors 8 along the respective linear axis 28a or 28b offers a first optical measurement setup for obtaining a self-calibrated LPDC signal and a second optical measurement setup for obtaining a self-calibrated DPDC signal. In other words, each design according to figure 8 / 9 / 10 offers a total of two OMS1 13 and two OMS2 14, i.e. a total of four self-calibrated optical measurement setups.

For example, in the design of figure 8, the detectors 8a and 8b, are linearly arranged with respect to both axes 28a and 28b. Hence, detectors 8a and 8b are employed in all four OMS.

In the design according to figure 10, there are a total of four innermost detectors 8a, 8b, 8c and 8d. Detectors 8a and 8d are used in the linear arrangement along axis 28b, while detectors 8c and 8b are employed in the linear arrangement along axis 28a.

Using such X-designs, as displayed in figures 8-10 offers the advantage of enabling a mapping of self-calibrated LPDC and DPDC signals.

In all of the designs according to figures 8-11, the light sources 7 and detectors 8 forming the respective OMS1 or OMS2 are respectively arranged linearly along a longitudinal axis 28. This is also true for the designs according to figures 8-10, with respect to the respective longitudinal axis 28a or 28b.

Further functionality may be added to the apparatus 5 by providing two more sources 7 or two more detectors 8, as illustrated in figure 13. The design of figure 13 is similar to that of figure 7, but two more detectors D3 and D4 have been added on the longitudinal axis 28 along with a fifth detector D5 in the center of the arrangement. The two further detectors D3 and D4 can be used to form a third and fourth OMS3 and OMS4, similar to OMS1, but shifted to the left and to the right with respect to the center 19 of the arrangement. This approach allows computation of two more self-calibrated LPDC signals, which represent data obtained from superficial layers on the left and right end of the arrangement, as compared to OMS1, which optically probes superficial tissue layers in the center of the arrangement. By comparing these 3 LPDC signals, a robust optical contact mapping scheme and even some coarse kind of spatial imaging of properties of these superficial layers may be achieved.

The additional detector D5 on the symmetry line in the middle of OMS1 offers the advantage that a mismatch of the optical properties measured in superficial layers under the left and right part of inner OMS1 can be detected. This may be used for checking if the apparatus is closely attached onto the skin over the complete measurement area.

In summary, for improving the accuracy and precision of measurements of optical or physiological parameters in a scattering medium 6 such as human tissue, an apparatus 5 is proposed featuring two optical measurements sets, OMS1 13 and OMS2 14, each comprising several light sources 7 and detectors 8 defining two pairs of substantially equal source-detector-separations, 1a/1b, 2a/2b, and 3a/3b, 4a/4b, respectively, wherein OMS2 14 offers larger source-detector-separations as OMS1 13, and wherein an electronic unit 11 of the apparatus 5 is configured to compute self-calibrated measurement signals from detector signals provided by OMS1 13 and OMS2 14, respectively, which are representative of measurements of said optical parameter at different depth in the medium 6 and which each require no calibration prior to the measurement (c.f. figure 4).

### List of reference numerals

- 1: first source-detector-separations
- 2: second source-detector-separations
- 3: third source-detector-separations
- 4: fourth source-detector-separations
- 5: apparatus
- 6: scattering medium (e.g. tissue)
- 7: light source
- 8: detector
- 9: source set
- 10: detector set
- 11: electronic unit
- 12: measurement surface
- 13: first optical measurement set
- 14: second optical measurement set
- 15: two spaced detectors (of 13)
- 16: two spaced detectors (of 14)
- 17: circle
- 18: diameter (of 17)
- 19: center (of 17)
- 20: two spaced sources (of 13)
- 21: source sub-set
- 22: detector sub-set
- 23: two spaced sources (of 14)
- 24: global symmetry line
- 25: local symmetry line
- 26: output unit
- 27: computing unit
- 28: longitudinal axis
- 29: hull
- 30: proximal end
- 31: distal end
- 32: measurement printed circuit board
- 33: printed circuit board
- 34: flat cable
- 35: window
- 36: optical barrier
- 37: slit aperture
- 38: skin
- 39: mean optical path
- 40: contact surface
- 41: sensor head
- 42: intersection point

## Claims

1. **Apparatus (5)** for measuring at least one optical or physiological parameter in a scattering medium (6), comprising
- a source set (9) consisting of at least two light sources (7) spaced from each other and each configured to emit a measurement wavelength, wherein said measurement wavelengths are substantially equal,
- a detector set (10) consisting of at least two detectors (8) spaced from each other and each configured to detect said measurement wavelengths after being backscattered by said medium (6), and
- an electronic unit (11) configured to read-out said at least two detectors (8), wherein
- said at least two light sources (7) and said at least two detectors (8) are arranged on a measurement surface (12) such that
- a first optical measurement set OMS1 (13) is formed, featuring two first source-detector-separations (1a, 1b) substantially equal in size along with two second source-detector-separations (2a, 2b) substantially equal in size and each larger than each of said first source-detector-separations (2a or 2b > 1a or 1b),
wherein
- a second optical measurement set OMS2 (14) is formed on said measurement surface (12), featuring two third source-detector-separations (3a, 3b) substantially equal in size along with two fourth source-detector-separations (4a, 4b) substantially equal in size and each larger than each of said third source-detector-separations (4a or 4b > 3a or 3b),
- wherein said fourth source-detector-separations are larger than said second source-detector-separations (4a or 4b > 2a or 2b), and wherein
the two optical measurement sets OMS1 (13) and OMS2 (14) each feature two spaced detectors D1, D2, D3, D4 (8) and wherein the two spaced detectors D2, D3 (8) of OMS1 (13) lie within an area enclosed by a circle
- whose diameter is a distance between the two spaced detectors D1, D4 (8) of OMS2 (14) and
- whose center is equidistant from said at least two spaced detectors D1, D4 (8) of OMS2 (14)
or
the two optical measurement sets OMS1 (13) and OMS2 (14) each feature two spaced sources S1, S2, S3, S4 (7) and wherein the two spaced sources S2, S3 (7) of OMS1 (13) lie within an area enclosed by a circle (17)
- whose diameter is a distance between the two spaced sources S1, S4 (7) of OMS2 (14) and
- whose center is equidistant from said at least two sources S1, S4 (7) of OMS2 (14),
- and wherein
- OMS1 (13) is located such that a first region in said medium (6) optically probed by OMS1 (13) lies within a second region probed by OMS2 (14) and
- OMS1 (13) forms an inner optical measurement set with respect to OMS2 (14),
and
- wherein said electronic unit (11) is configured
- to evaluate measurement signals, obtained from OMS1 (13) in a self-calibrating-scheme and to output a self-calibrated low penetration depth channel (scLPDC) signal obtained from OMS1 (13), and
- to evaluate measurement signals, obtained from OMS2 (14) in a self-calibrating-scheme and to output a self-calibrated deep penetration depth channel (scDPDC) signal obtained from OMS2 (14);
**characterized in that**
the sources (7) and detectors (8) forming the respective OMS1 (13) and OMS2 (14), respectively, are each linearly arranged on a respective longitudinal axis (28a, 28b) and these two longitudinal axes (28a, 28b) either coincide or are arranged in an X-formation featuring an intersection point (42).

2. Apparatus (5) according to claim 1,
- wherein said apparatus is configured to output an early warning signal, in case a measurement quantity computed by the electronic unit from said scLPDC signal differs beyond a threshold value from a measurement quantity computed by the electronic unit from said scDPDC signal,
- in particular wherein the apparatus (5) is configured to display a chronological sequence of these two measurement quantities computed from said scLPDC signal and said scDPDC signal, respectively.

3. Apparatus (5) according to claim 1 or 2,
- wherein said first source-detector-separations are shorter than or substantially equal to said third source-detector-separations (1a or 1b ≤ 3a or 3b),
- wherein said third source-detector-separations are substantially equal to said second source-detector-separations (3a or 3b ≈ 2a or 2b) or
- wherein said third source-detector-separations are substantially longer than said second source-detector-separations (3a or 3b > 2a or 2b) or
- wherein said third source-detector-separations are substantially shorter than said second source-detector-separations (3a or 3b < 2a or 2b).

4. Apparatus (5) according to one of the preceding claims, wherein OMS1 (13) and OMS2 (14) each comprise
- a source sub-set (21) consisting of at least two light sources (7) spaced from each other, and
- a detector sub-set (22) consisting of at least two detectors (8) spaced from each other, and
- wherein said at least two light sources (7) of said source sub-sets (21) and said at least two detectors (8) of said detector sub-sets (22) are each symmetrically arranged to a common global symmetry line (24),
- preferably wherein at least one source-detector-pair defining one of said two second source-detector-separations (2a, 2b) and at least one source-detector-pair defining one of said two third source-detector- separations (3a, 3b) are symmetrically arranged to a respective common local symmetry line (25).

5. Apparatus (1) according to one of the preceding claims,
- wherein said second source-detector-separations (2a, 2b) and/or said first source-detector-separations (1a, 1b) are shorter than 12 mm, most preferably shorter than 10 mm,
- in particular wherein said fourth source-detector-separations (4a, 4b) are longer than 20 mm, most preferably longer than 25 mm,
- in particular such that signal components resulting from superficial tissue in the head of an adult can be regressed from said scDPDC signal obtained from OMS2 (14) by taking into account said scLPDC signal obtained from OMS1 (13).

6. Apparatus (5) according to one of the preceding claims,
- wherein said second source-detector-separations (2a, 2b) and/or said first source-detector-separations (1a, 1b) are shorter than 5 mm, most preferably shorter than 3 mm,
- in particular wherein said fourth source-detector-separations (4a, 4b) are longer than 15 mm, most preferably longer than 20 mm.

7. Apparatus (5) according to one of the preceding claims,
- wherein said first source-detector-separations (1a, 1b) are between 1 and 15 mm,
- said second and third source-detector-separations (2a, 2b, 3a, 3b) are between 10 mm and 25 mm and
- said fourth source-detector-separations (4a, 4b) are between 20 mm and 65 mm.

8. Apparatus (5) according to one of the preceding claims,
- wherein said apparatus (5) comprises an output unit (26) configured to output, for example acoustically and/or optically, a first measurement value, in particular a first concentration of a chromophore in said medium or a first StO2-value, derived from said scDPDC signal obtained from OMS2 (14) and measured in a deep region of said medium (6),
- preferably and to output a second measurement value, in particular a second concentration of said chromophore in said medium or a second StO2-value, derived from said scLPDC signal obtained from OMS1 (13) and measured in a superficial region of said medium (6), located between said deep region and said measurement surface (12).

9. Apparatus (5) according to claim 8,
- wherein said output unit (26) is configured to output, for example acoustically and/or optically, preferably along with said first measurement value, a first MPVD-value displaying an MPVD probed by OMS2 (14),
- preferably and to output, preferably along with said second measurement value, a second MPVD-value displaying an MPVD probed by OMS1 (13).

10. Apparatus (5) according to one of the preceding claims,
- wherein said apparatus (5) comprises a computing unit (27), which may be part of said electronic unit (11),
- configured to regress superficial components from said scDPDC obtained from OMS2 (14) by processing said scDPDC and said scLPDC signal obtained from OMS1 (13) and to
- deliver a corrected deep penetration depth channel (cDPDC) signal that is substantially free of optical noise arising from superficial layers optically probed by OMS1 (13),
- in particular wherein an output unit (26) of said apparatus (5), in particular said output unit (26), is configured to output, for example acoustically and/or optically, a corrected measurement value of said parameter computed from said scDPDC signal.

11. Apparatus (5) according to one of the preceding claims,
- wherein said apparatus (5) comprises a computing unit (27), which may be said computing unit (27), configured to compute a physiological parameter from said scLPDC signal obtained from said OMS1 (13),
- preferably by applying a pulse oximetry measurement scheme,
- in particular wherein said apparatus (5) features an analog or digital filter configured to separate an AC-component from said scLPDC signal and said computing unit is configured to compute a temporal variation of said concentration from said AC-component, and/or
- wherein said apparatus (5) features an amplifier with an adjustable frequency transfer function, for example by way of a control voltage, and said computing unit is configured to adjust said frequency transfer function to separate an AC-component from an amplified signal outputted by said amplifier and to compute said concentration from said AC-component of said amplified signal.

12. Apparatus (5) according to one of the preceding claims,
- wherein said measurement wavelengths lie in the NIR-range, i.e. between 780 nm and 2500 nm and/or
- wherein said source set (9) emits at least two, preferably four, most preferably eight different measurement wavelengths,
- preferably wherein said at least two, preferably four, most preferably eight different measurement wavelengths are separated from each other by at least 10 nm, preferably by at least 20 nm, and/or
- comprise at least one wavelength in the UV-VIS-range, i.e. between 100 nm and 780 nm, and/or
- comprise at least one wavelength beyond 830 nm.

13. **Method for obtaining a corrected deep penetration depth channel (cDPDC) signal**
- using an apparatus (5) according to one of the preceding claims designed for measuring an optical or physiological parameter in a scattering medium (6)
- by illuminating the medium (6) with at least one measurement wavelength and
- recording at least one intensity of backscattered light at the measurement wavelength with at least one detector (7) of said apparatus (5),
the method comprising the steps of:
- acquiring a low penetration depth channel signal (LPDC) signal with a first optical measurement setup OMS1 (13) of the apparatus (5) offering two pairs of substantially equal source-detector-separations (1a/1b and 2a/2b), wherein the two pairs of the OMS1 (13) differ in their source-detector-separation (SDS) values,
- acquiring a deep penetration depth channel signal (DPDC) with a second optical measurement setup OMS2 (14) of the apparatus (5) offering two pairs of substantially equal SDS (3a/3b and 4a/4b), wherein the two pairs of the OMS2 (14) differ in their SDS values, and wherein OMS2 (14) offers larger SDS values as OMS1 (13),
- applying a self-calibration algorithm on the LPDC signal and the DPDC signal, respectively, to obtain a self-calibrated low penetration depth channel (scLPDC) signal and a self-calibrated deep penetration depth channel (scDPDC) signal, and
- regressing signal components of the scLPDC signal from the scDPDC signal to obtain a cDPDC signal.

## Patentansprüche

1. Vorrichtung (5) zum Messen mindestens eines optischen oder physiologischen Parameters in einem streuenden Medium (6), umfassend
- einen Quellensatz (9), bestehend aus mindestens zwei Lichtquellen (7), die voneinander beabstandet sind und jeweils dafür konfiguriert sind, eine Messwellenlänge auszusenden, wobei die Messwellenlängen im Wesentlichen gleich sind,
- einen Detektorsatz (10), bestehend aus mindestens zwei Detektoren (8), die voneinander beabstandet sind und jeweils dafür konfiguriert sind, die Messwellenlängen zu detektieren, nachdem sie durch das Medium (6) rückgestreut wurden, und
- eine elektronische Einheit (11), die dafür konfiguriert ist, die mindestens zwei Detektoren (8) auszulesen, wobei
- die mindestens zwei Lichtquellen (7) und die mindestens zwei Detektoren (8) auf einer Messoberfläche (12) so angeordnet sind, dass
- ein erster optischer Messsatz OMS1 (13) gebildet ist, der zwei erste Quellen-Detektor-Abstände (1a, 1b) von im Wesentlichen gleicher Größe sowie zwei zweite Quellen-Detektor-Abstände (2a, 2b) von im Wesentlichen gleicher Größe aufweist, die jeweils größer als jeder der ersten Quellen-Detektor-Abstände sind (2a oder 2b > 1a oder 1b) wobei
- ein zweiter optischer Messsatz OMS2 (14) auf der Messoberfläche (12) gebildet ist, der zwei dritte Quellen-Detektor-Abstände (3a, 3b) von im Wesentlichen gleicher Größe sowie zwei vierte Quellen-Detektor-Abstände (4a, 4b) von im Wesentlichen gleicher Größe aufweist, die jeweils größer als jeder der dritten Quellen-Detektor-Abstände sind (4a oder 4b > 3a oder 3b),
- wobei die vierten Quellen-Detektor-Abstände größer als die zweiten Quellen-Detektor-Abstände sind (4a oder 4b > 2a oder 2b) und wobei
die beiden optischen Messsätze OMS1 (13) und OMS2 (14) jeweils zwei beabstandete Detektoren D1, D2, D3, D4 (8) aufweisen und wobei die beiden beabstandeten Detektoren D2, D3 (8) von OMS1 (13) innerhalb einer Fläche liegen, die von einem Kreis umschlossen ist,
- dessen Durchmesser eine Entfernung zwischen den beiden beabstandeten Detektoren D1, D4 (8) von OMS2 (14) ist und
- dessen Mittelpunkt gleich weit von den mindestens zwei beabstandeten Detektoren D1, D4 (8) von OMS2 (14) entfernt ist
oder
die beiden optischen Messsätze OMS1 (13) und OMS2 (14) jeweils zwei voneinander beabstandete Quellen S1, S2, S3, S4 (7) aufweisen und wobei die beiden voneinander beabstandeten Quellen S2, S3 (7) von OMS1 (13) innerhalb einer Fläche liegen, die von einem Kreis (17) umschlossen ist,
- dessen Durchmesser eine Entfernung zwischen den beiden voneinander beabstandeten Quellen S1, S4 (7) von OMS2 (14) ist und
- dessen Mittelpunkt gleich weit von den mindestens zwei Quellen S1, S4 (7) von OMS2 (14) entfernt ist,
- und wobei
- OMS1 (13) so angeordnet ist, dass ein erster Bereich in dem Medium (6), der von OMS1 (13) optisch untersucht wird, innerhalb eines zweiten Bereichs liegt, der von OMS2 (14) untersucht wird, und
- OMS1 (13) in Bezug auf OMS2 (14) einen inneren optischen Messsatz bildet, und
- wobei die elektronische Einheit (11) dafür konfiguriert ist,
- Messsignale, die von OMS1 (13) in einem Selbstkalibrierungsschema erhalten wurden, auszuwerten und ein selbstkalibriertes Signal des Kanals mit geringer Eindringtiefe (scLPDC) auszugeben, das von OMS1 (13) erhalten wird, und
- Messsignale, die von OMS2 (14) in einem Selbstkalibrierungsschema erhalten wurden, auszuwerten und ein selbstkalibriertes Signal des Kanals mit tiefer Eindringtiefe (scDPDC) auszugeben, das von OMS2 (14) erhalten wird;
**dadurch gekennzeichnet, dass**
die Quellen (7) und Detektoren (8), die die jeweiligen OMS1 (13) bzw. OMS2 (14) bilden, jeweils linear auf einer jeweiligen Längsachse (28a, 28b) angeordnet sind und diese beiden Längsachsen (28a, 28b) entweder zusammenfallen oder in einer X-Formation angeordnet sind, die einen Schnittpunkt (42) aufweist.

2. Vorrichtung (5) nach Anspruch 1,
- wobei die Vorrichtung dafür konfiguriert ist, ein Frühwarnsignal auszugeben, falls eine Messgröße, die von der elektronischen Einheit aus dem scLPDC-Signal berechnet wird, über einen Schwellenwert hinaus von einer Messgröße abweicht, die von der elektronischen Einheit aus dem scDPDC-Signal berechnet wird,
- insbesondere wobei die Vorrichtung (5) dafür konfiguriert ist, eine chronologische Abfolge dieser beiden Messgrößen anzuzeigen, die jeweils aus dem scLPDC-Signal und dem scDPDC-Signal berechnet werden.

3. Vorrichtung (5) nach Anspruch 1 oder 2,
- wobei die ersten Quellen-Detektor-Abstände kürzer als die dritten Quellen-Detektor-Abstände oder im Wesentlichen gleich diesen sind (1a oder 1b ≤ 3a oder 3b),
- wobei die dritten Quellen-Detektor-Abstände im Wesentlichen gleich den zweiten Quellen-Detektor-Abständen sind (3a oder 3b ≈ 2a oder 2b) oder
- wobei die dritten Quellen-Detektor-Abstände im Wesentlichen länger als die zweiten Quellen-Detektor-Abstände sind (3a oder 3b > 2a oder 2b) oder
- wobei die dritten Quellen-Detektor-Abstände im Wesentlichen kürzer als die zweiten Quellen-Detektor-Abstände sind (3a oder 3b < 2a oder 2b).

4. Vorrichtung (5) nach einem der vorhergehenden Ansprüche, wobei OMS1 (13) und OMS2 (14) jeweils Folgendes umfassen:
- eine Quellenuntergruppe (21), bestehend aus mindestens zwei voneinander beabstandeten Lichtquellen (7), und
- eine Detektoruntergruppe (22), bestehend aus mindestens zwei voneinander beabstandeten Detektoren (8), und
- wobei die mindestens zwei Lichtquellen (7) der Quellenuntergruppen (21) und die mindestens zwei Detektoren (8) der Detektoruntergruppen (22) jeweils symmetrisch zu einer gemeinsamen globalen Symmetrielinie (24) angeordnet sind,
- vorzugsweise wobei mindestens ein Quellen-Detektor-Paar, das einen der beiden zweiten Quellen-Detektor-Abstände (2a, 2b) definiert, und mindestens ein Quellen-Detektor-Paar, das einen der beiden dritten Quellen-Detektor-Abstände (3a, 3b) definiert, symmetrisch zu einer jeweiligen gemeinsamen lokalen Symmetrielinie (25) angeordnet sind.

5. Vorrichtung (1) nach einem der vorhergehenden Ansprüche,
- wobei die zweiten Quellen-Detektor-Abstände (2a, 2b) und/oder die ersten Quellen-Detektor-Abstände (1a, 1b) kürzer als 12 mm, am stärksten bevorzugt kürzer als 10 mm sind,
- insbesondere wobei die vierten Quellen-Detektor-Abstände (4a, 4b) länger als 20 mm, am stärksten bevorzugt länger als 25 mm sind,
- insbesondere derart, dass Signalkomponenten, die aus oberflächlichem Gewebe im Kopf eines Erwachsenen resultieren, aus dem von OMS2 (14) erhaltenen scDPDC-Signal regrediert werden können, indem das von OMS1 (13) erhaltene scLPDC-Signal berücksichtigt wird.

6. Vorrichtung (5) nach einem der vorhergehenden Ansprüche,
- wobei die zweiten Quellen-Detektor-Abstände (2a, 2b) und/oder die ersten Quellen-Detektor-Abstände (1a, 1b) kürzer als 5 mm, am stärksten bevorzugt kürzer als 3 mm sind,
- insbesondere wobei die vierten Quellen-Detektor-Abstände (4a, 4b) länger als 15 mm, am stärksten bevorzugt länger als 20 mm sind.

7. Vorrichtung (5) nach einem der vorhergehenden Ansprüche,
- wobei die ersten Quellen-Detektor-Abstände (1a, 1b) zwischen 1 und 15 mm betragen,
- wobei die zweiten und dritten Quellen-Detektor-Abstände (2a, 2b, 3a, 3b) zwischen 10 mm und 25 mm betragen und
- wobei die vierten Quellen-Detektor-Abstände (4a, 4b) zwischen 20 mm und 65 mm betragen.

8. Vorrichtung (5) nach einem der vorhergehenden Ansprüche,
- wobei die Vorrichtung (5) eine Ausgabeeinheit (26) umfasst, die dafür konfiguriert ist, beispielsweise akustisch und/oder optisch einen ersten Messwert auszugeben, insbesondere eine erste Konzentration eines Chromophors in dem Medium oder einen ersten StO2-Wert, der aus dem von OMS2 (14) erhaltenen scDPDC-Signal abgeleitet und in einem tiefen Bereich des Mediums (6) gemessen wird,
- und vorzugsweise einen zweiten Messwert auszugeben, insbesondere eine zweite Konzentration des Chromophors in dem Medium oder einen zweiten StO2-Wert, der aus dem von OMS1 (13) erhaltenen scLPDC-Signal abgeleitet und in einem oberflächlichen Bereich des Mediums (6) gemessen wird, der sich zwischen dem tiefen Bereich und der Messoberfläche (12) befindet.

9. Vorrichtung (5) nach Anspruch 8,
- wobei die Ausgabeeinheit (26) dafür konfiguriert ist, beispielsweise akustisch und/oder optisch, vorzugsweise zusammen mit dem ersten Messwert, einen ersten MPVD-Wert auszugeben, der eine von OMS2 (14) untersuchte MPVD anzeigt,
- und vorzugsweise zusammen mit dem zweiten Messwert einen zweiten MPVD-Wert auszugeben, der eine von OMS1 (13) untersuchte MPVD anzeigt.

10. Vorrichtung (5) nach einem der vorhergehenden Ansprüche,
- wobei die Vorrichtung (5) eine Recheneinheit (27) umfasst, die Teil der elektronischen Einheit (11) sein kann,
- die dafür konfiguriert ist, Oberflächenkomponenten aus dem von OMS2 (14) erhaltenen scDPDC zu regredieren, indem das scDPDC und das von OMS1 (13) erhaltene scLPDC-Signal verarbeitet werden und
- ein korrigiertes Signal des Kanals mit tiefer Eindringtiefe (cDPDC) zu liefern, das im Wesentlichen frei von optischem Rauschen ist, das von oberflächlichen Schichten stammt, die durch OMS1 (13) optisch untersucht werden,
- insbesondere wobei eine Ausgabeeinheit (26) der Vorrichtung (5), insbesondere die Ausgabeeinheit (26), dafür konfiguriert ist, beispielsweise akustisch und/oder optisch einen korrigierten Messwert des Parameters auszugeben, der aus dem scDPDC-Signal berechnet wird.

11. Vorrichtung (5) nach einem der vorhergehenden Ansprüche,
- wobei die Vorrichtung (5) eine Recheneinheit (27) umfasst, die die Recheneinheit (27) sein kann und dafür konfiguriert ist, einen physiologischen Parameter aus dem von OMS1 (13) erhaltenen scLPDC-Signal zu berechnen,
- vorzugsweise durch Anwendung eines Pulsoximetrie-Messschemas,
- insbesondere wobei die Vorrichtung (5) einen analogen oder digitalen Filter aufweist, der dafür konfiguriert ist, eine Wechselstromkomponente von dem scLPDC-Signal zu trennen, und wobei die Recheneinheit dafür konfiguriert ist, eine zeitliche Veränderung der Konzentration aus der Wechselstromkomponente zu berechnen, und/oder
- wobei die Vorrichtung (5) einen Verstärker mit einer einstellbaren Frequenzübertragungsfunktion, beispielsweise mittels einer Steuerspannung, aufweist und wobei die Recheneinheit dafür konfiguriert ist, die Frequenzübertragungsfunktion einzustellen, um eine Wechselstromkomponente von einem vom Verstärker ausgegebenen verstärkten Signal zu trennen und die Konzentration aus der Wechselstromkomponente des verstärkten Signals zu berechnen.

12. Vorrichtung (5) nach einem der vorhergehenden Ansprüche,
- wobei die Messwellenlängen im NIR-Bereich liegen, d. h. zwischen 780 nm und 2500 nm, und/oder
- wobei der Quellensatz (9) mindestens zwei, vorzugsweise vier, am stärksten bevorzugt acht verschiedene Messwellenlängen aussendet,
- vorzugsweise wobei die mindestens zwei, vorzugsweise vier, am stärksten bevorzugt acht verschiedenen Messwellenlängen um mindestens 10 nm, vorzugsweise um mindestens 20 nm voneinander getrennt sind, und/oder
- mindestens eine Wellenlänge im UV-VIS-Bereich, d. h. zwischen 100 nm und 780 nm umfassen, und/oder
- mindestens eine Wellenlänge über 830 nm umfassen.

13. **Verfahren zum Erhalten eines korrigierten Signals des Kanals mit tiefer Eindringtiefe (cDPDC)**
- unter Verwendung einer Vorrichtung (5) nach einem der vorhergehenden Ansprüche, die zum Messen eines optischen oder physiologischen Parameters in einem streuenden Medium (6) ausgelegt ist,
- durch Beleuchten des Mediums (6) mit mindestens einer Messwellenlänge und
- Aufzeichnen mindestens einer Intensität des rückgestreuten Lichts bei der Messwellenlänge mit mindestens einem Detektor (7) der Vorrichtung (5), wobei das Verfahren die folgenden Schritte umfasst:
- Erfassen eines Signals des Kanals mit geringer Eindringtiefe (LPDC) mit einer ersten optischen Messeinrichtung OMS1 (13) der Vorrichtung (5), die zwei Paare von im Wesentlichen gleichen Quellen-Detektor-Abständen (1a/1b und 2a/2b) bietet, wobei sich die beiden Paare der OMS1 (13) in ihren Werten für die Quellen-Detektor-Abstände (SDS) unterscheiden,
- Erfassen eines Signals des Kanals mit tiefer Eindringtiefe (DPDC) mit einer zweiten optischen Messeinrichtung OMS2 (14) der Vorrichtung (5), die zwei Paare von im Wesentlichen gleichen SDS (3a/3b und 4a/4b) bietet, wobei sich die beiden Paare der OMS2 (14) in ihren SDS-Werten unterscheiden und wobei OMS2 (14) größere SDS-Werte als OMS1 (13) bietet,
- Anwenden eines Selbstkalibrierungsalgorithmus auf das LPDC-Signal bzw. das DPDC-Signal, um ein selbstkalibriertes Signal des Kanals mit geringer Eindringtiefe (scLPDC) und ein selbstkalibriertes Signal des Kanals mit tiefer Eindringtiefe (scDPDC) zu erhalten, und
- Regredieren von Signalkomponenten des scLPDC-Signals aus dem scDPDC-Signal, um ein cDPDC-Signal zu erhalten.

## Revendications

1. **Appareil (5)** pour mesurer au moins un paramètre optique ou physiologique dans un milieu diffusant (6), comprenant
- un ensemble de sources (9) consistant en au moins deux sources de lumière (7) espacées l'une de l'autre et configurées chacune pour émettre une longueur d'onde de mesure, dans lequel lesdites longueurs d'onde de mesure sont sensiblement égales,
- un ensemble de détecteurs (10) consistant en au moins deux détecteurs (8) espacés l'un de l'autre et configurés chacun pour détecter lesdites longueurs d'onde de mesure après qu'elles ont été rétrodiffusées par ledit milieu (6), et
- une unité électronique (11) configurée pour lire lesdits au moins deux détecteurs (8), dans lequel
- lesdites au moins deux sources de lumière (7) et lesdits au moins deux détecteurs (8) sont agencés sur une surface de mesure (12) de telle sorte que
- un premier ensemble de mesure optique OMS1 (13) est formé, présentant deux premières séparations source-détecteur (1a, 1b) de taille sensiblement égale, ainsi que deux deuxièmes séparations source-détecteur (2a, 2b) de taille sensiblement égale et chacune plus grande que chacune desdites premières séparations source-détecteur (2a ou 2b > 1a ou 1b), dans lequel
- un deuxième ensemble de mesure optique OMS2 (14) est formé sur ladite surface de mesure (12), présentant deux troisièmes séparations source-détecteur (3a, 3b) de taille sensiblement égale, ainsi que deux quatrièmes séparations source-détecteur (4a, 4b) de taille sensiblement égale et chacune plus grande que chacune desdites troisièmes séparations source-détecteur (4a ou 4b > 3a ou 3b),
- dans lequel lesdites quatrièmes séparations source-détecteur sont plus grandes que lesdites deuxièmes séparations source-détecteur (4a ou 4b > 2a ou 2b), et dans lequel
les deux ensembles de mesure optique OMS1 (13) et OMS2 (14) présentent chacun deux détecteurs espacés D1, D2, D3, D4 (8) et dans lequel les deux détecteurs espacés D2, D3 (8) de l'OMS1 (13) se trouvent dans une zone délimitée par un cercle
- dont le diamètre est une distance entre les deux détecteurs espacés D1, D4 (8) de l'OMS2 (14) et
- dont le centre est équidistant desdits au moins deux détecteurs espacés D1, D4 (8) de l'OMS2 (14)
ou
les deux ensembles de mesure optique OMS1 (13) et OMS2 (14) présentent chacun deux sources espacées S1, S2, S3, S4 (7) et dans lequel les deux sources espacées S2, S3 (7) de l'OMS1 (13) se trouvent dans une zone délimitée par un cercle (17)
- dont le diamètre est une distance entre les deux sources espacées S1, S4 (7) de l'OMS2 (14) et
- dont le centre est équidistant desdites au moins deux sources S1, S4 (7) de l'OMS2 (14),
- et dans lequel
- l'OMS1 (13) est situé de telle sorte qu'une première région dans ledit milieu (6) sondée optiquement par l'OMS1 (13) se trouve au sein d'une deuxième région sondée par l'OMS2 (14) et
- l'OMS1 (13) forme un ensemble de mesure optique interne par rapport à l'OMS2 (14), et
- dans lequel ladite unité électronique (11) est configurée
- pour évaluer des signaux de mesure, obtenus à partir de l'OMS1 (13) dans un schéma d'autoétalonnage et pour délivrer en sortie un signal de canal à faible profondeur de pénétration autoétalonné (scLPDC) obtenu à partir de l'OMS1 (13), et
- pour évaluer des signaux de mesure, obtenus à partir de l'OMS2 (14) dans un schéma d'autoétalonnage et pour délivrer en sortie un signal de canal à profondeur de pénétration profonde autoétalonné (scDPDC) obtenu à partir de l'OMS2 (14) ;
**caractérisé en ce que**
les sources (7) et les détecteurs (8) formant les OMS1 (13) et OMS2 (14) respectifs, respectivement, sont chacun agencés linéairement sur un axe longitudinal (28a, 28b) respectif et ces deux axes longitudinaux (28a, 28b) soit coïncident soit sont agencés en une présentation de formation en X avec un point d'intersection (42).

2. Appareil (5) selon la revendication 1,
- dans lequel ledit appareil est configuré pour délivrer en sortie un signal d'alerte précoce, au cas où une quantité de mesure calculée par l'unité électronique à partir dudit signal scLPDC diffère au-delà d'une valeur seuil d'une quantité de mesure calculée par l'unité électronique à partir dudit signal scDPDC,
- en particulier dans lequel l'appareil (5) est configuré pour afficher une séquence chronologique de ces deux quantités de mesure calculées à partir dudit signal scLPDC et dudit signal scDPDC, respectivement.

3. Appareil (5) selon la revendication 1 ou 2,
- dans lequel lesdites premières séparations source-détecteur sont plus courtes ou sensiblement égales auxdites troisièmes séparations source-détecteur (1a ou 1b ≤ 3a ou 3b),
- dans lequel lesdites troisièmes séparations source-détecteur sont sensiblement égales auxdites deuxièmes séparations source-détecteur (3a ou 3b ≈ 2a ou 2b) ou
- dans lequel lesdites troisièmes séparations source-détecteur sont sensiblement plus longues que lesdites deuxièmes séparations source-détecteur (3a ou 3b > 2a ou 2b) ou
- dans lequel lesdites troisièmes séparations source-détecteur sont sensiblement plus courtes que lesdites deuxièmes séparations source-détecteur (3a ou 3b < 2a ou 2b).

4. Appareil (5) selon l'une des revendications précédentes, dans lequel l'OMS1 (13) et l'OMS2 (14) comprennent chacun
- un sous-ensemble de sources (21) consistant en au moins deux sources de lumière (7) espacées l'une de l'autre, et
- un sous-ensemble de détecteurs (22) consistant en au moins deux détecteurs (8) espacés l'un de l'autre, et
- dans lequel lesdites au moins deux sources de lumière (7) desdits sous-ensembles de sources (21) et lesdits au moins deux détecteurs (8) desdits sous-ensembles de détecteurs (22) sont chacun agencés symétriquement par rapport à une ligne de symétrie globale commune (24),
- de préférence dans lequel au moins une paire source-détecteur définissant l'une desdites deux deuxièmes séparations source-détecteur (2a, 2b) et au moins une paire source-détecteur définissant l'une desdites deux troisièmes séparations source-détecteur (3a, 3b) sont agencées symétriquement par rapport à une ligne de symétrie locale commune respective (25).

5. Appareil (1) selon l'une des revendications précédentes,
- dans lequel lesdites deuxièmes séparations source-détecteur (2a, 2b) et/ou lesdites premières séparations source-détecteur (1a, 1b) sont plus courtes que 12 mm, le plus préférentiellement plus courtes que 10 mm,
- en particulier dans lequel lesdites quatrièmes séparations source-détecteur (4a, 4b) sont plus longues que 20 mm, le plus préférentiellement plus longues que 25 mm,
- en particulier de telle sorte que des composantes de signal résultant de tissus superficiels dans la tête d'un adulte peuvent être régressées à partir dudit signal scDPDC obtenu à partir de l'OMS2 (14) en tenant compte dudit signal scLPDC obtenu à partir de l'OMS1 (13).

6. Appareil (5) selon l'une des revendications précédentes,
- dans lequel lesdites deuxièmes séparations source-détecteur (2a, 2b) et/ou lesdites premières séparations source-détecteur (1a, 1b) sont plus courtes que 5 mm, le plus préférentiellement plus courtes que 3 mm,
- en particulier dans lequel lesdites quatrièmes séparations source-détecteur (4a, 4b) sont plus longues que 15 mm, le plus préférentiellement plus longues que 20 mm.

7. Appareil (5) selon l'une des revendications précédentes,
- dans lequel lesdites premières séparations source-détecteur (1a, 1b) sont entre 1 et 15 mm,
- lesdites deuxième et troisième séparations source-détecteur (2a, 2b, 3a, 3b) sont entre 10 mm et 25 mm et
- lesdites quatrièmes séparations source-détecteur (4a, 4b) sont entre 20 mm et 65 mm.

8. Appareil (5) selon l'une des revendications précédentes,
- dans lequel ledit appareil (5) comprend une unité de sortie (26) configurée pour délivrer en sortie, par exemple de manière acoustique et/ou optique, une première valeur de mesure, en particulier une première concentration d'un chromophore dans ledit milieu ou une première valeur St02, dérivée dudit signal scDPDC obtenu à partir de l'OMS2 (14) et mesurée dans une région profonde dudit milieu (6),
- de préférence et pour délivrer en sortie une deuxième valeur de mesure, en particulier une deuxième concentration dudit chromophore dans ledit milieu ou une deuxième valeur St02, dérivée dudit signal scLPDC obtenu à partir de l'OMS1 (13) et mesurée dans une région superficielle dudit milieu (6), située entre ladite région profonde et ladite surface de mesure (12).

9. Appareil (5) selon la revendication 8,
- dans lequel ladite unité de sortie (26) est configurée pour délivrer en sortie, par exemple de manière acoustique et/ou optique, de préférence avec ladite première valeur de mesure, une première valeur MPVD affichant une MPVD sondée par l'OMS2 (14),
- de préférence et pour délivrer en sortie, de préférence avec ladite deuxième valeur de mesure, une deuxième valeur MPVD affichant une MPVD sondée par l'OMS1 (13).

10. Appareil (5) selon l'une des revendications précédentes,
- dans lequel ledit appareil (5) comprend une unité de calcul (27), qui peut faire partie de ladite unité électronique (11),
- configurée pour régresser des composantes superficielles à partir dudit scDPDC obtenu à partir de l'OMS2 (14) en traitant ledit scDPDC et ledit signal scLPDC obtenu à partir de l'OMS1 (13) et pour
- délivrer un signal de canal à profondeur de pénétration profonde corrigé (cDPDC) qui est sensiblement exempt de bruit optique provenant des couches superficielles sondées optiquement par l'OMS1 (13),
- en particulier, dans lequel une unité de sortie (26) dudit appareil (5), en particulier ladite unité de sortie (26), est configurée pour délivrer en sortie, par exemple de manière acoustique et/ou optique, une valeur de mesure corrigée dudit paramètre calculé à partir dudit signal scDPDC.

11. Appareil (5) selon l'une des revendications précédentes,
- dans lequel ledit appareil (5) comprend une unité de calcul (27), qui peut être ladite unité de calcul (27), configurée pour calculer un paramètre physiologique à partir dudit signal scLPDC obtenu à partir dudit OMS1 (13),
- de préférence en appliquant un schéma de mesure par oxymétrie de pouls,
- en particulier dans lequel ledit appareil (5) présente un filtre analogique ou numérique configuré pour séparer une composante alternative dudit signal scLPDC et ladite unité de calcul est configurée pour calculer une variation temporelle de ladite concentration à partir de ladite composante alternative, et/ou
- dans lequel ledit appareil (5) présente un amplificateur avec une fonction de transfert de fréquence réglable, par exemple au moyen d'une tension de commande, et ladite unité de calcul est configurée pour régler ladite fonction de transfert de fréquence afin de séparer une composante alternative d'un signal amplifié délivré en sortie par ledit amplificateur et pour calculer ladite concentration à partir de ladite composante alternative dudit signal amplifié.

12. Appareil (5) selon l'une des revendications précédentes,
- dans lequel lesdites longueurs d'onde de mesure se situent dans la plage NIR, c'est-à-dire entre 780 nm et 2500 nm et/ou
- dans lequel ledit ensemble de sources (9) émet au moins deux, de préférence quatre, le plus préférentiellement huit longueurs d'onde de mesure différentes,
- de préférence dans lequel lesdites au moins deux, de préférence quatre, le plus préférentiellement huit longueurs d'onde de mesure différentes sont séparées les unes des autres d'au moins 10 nm, de préférence d'au moins 20 nm, et/ou
- comprennent au moins une longueur d'onde dans la plage UV-VIS, c'est-à-dire entre 100 nm et 780 nm, et/ou
- comprennent au moins une longueur d'onde au-delà de 830 nm.

13. **Procédé pour obtenir un signal de profondeur de pénétration profonde corrigé (cDPDC)**
- à l'aide d'un appareil (5) selon l'une des revendications précédentes conçu pour mesurer un paramètre optique ou physiologique dans un milieu diffusant (6)
- en illuminant le milieu (6) avec au moins une longueur d'onde de mesure et
- en enregistrant au moins une intensité de lumière rétrodiffusée à la longueur d'onde de mesure avec au moins un détecteur (7) dudit appareil (5),
le procédé comprenant les étapes suivantes :
- l'acquisition d'un signal de canal à faible profondeur de pénétration (LPDC) avec un premier dispositif de mesure optique OMS1 (13) de l'appareil (5) offrant deux paires de séparations source-détecteur sensiblement égales (1a/1b et 2a/2b), dans lequel les deux paires de l'OMS1 (13) diffèrent par leurs valeurs de séparation source-détecteur (SDS),
- l'acquisition d'un signal de canal à profondeur de pénétration profonde (DPDC) avec un deuxième dispositif de mesure optique OMS2 (14) de l'appareil (5) offrant deux paires de SDS sensiblement égales (3a/3b et 4a/4b), dans lequel les deux paires de l'OMS2 (14) diffèrent par leurs valeurs SDS, et dans lequel l'OMS2 (14) offre des valeurs SDS plus grandes que l'OMS1 (13),
- l'application d'un algorithme d'autoétalonnage au signal LPDC et au signal DPDC, respectivement, pour obtenir un signal de canal à faible profondeur de pénétration autoétalonné (scLPDC) et un signal de canal à profondeur de pénétration profonde autoétalonné (scDPDC), et
- la régression de composantes de signal du signal scLPDC à partir du signal scDPDC pour obtenir un signal cDPDC.
